# FASCICULE DE BREVET EUROPEEN

(11) **EP 3 096 691 B1**
(45) Date de publication et mention de la délivrance du brevet: **04.10.2023**
(21) Numéro de dépôt: 15703478.6
(22) Date de dépôt: 22.01.2015
(51) Int. Cl.: A61B 8/12, A61B 8/00, A61M 25/00, A61B 8/08

(54) **CATHETER**
KATHETER
CATHETER

(30) Priorité: 22.01.2014 FR 1450513
(43) Date de publication de la demande: 30.11.2016
(73) Titulaire: Labrousse, Louis, Marc, Jean, Lucien, 33000 Bordeaux (FR); Leroux, Lionel, Jean, Pierre, 33200 Bordeaux (FR)
(72) Inventeur: Labrousse, Louis, Marc, Jean, Lucien, 33000 Bordeaux (FR); Leroux, Lionel, Jean, Pierre, 33200 Bordeaux (FR)
(74) Mandataire: Cabinet Camus Lebkiri
(86) Numéro de dépôt international: PCT/EP2015/051190
(87) Numéro de publication internationale: WO 2015/110496

(56) Documents cités:
- US-A1- 2004 068 191
- US-A1- 2009 088 648
- US-A1- 2011 021 911

## Description

### DOMAINE

Le domaine de l'invention concerne les cathéters. Plus particulièrement, le domaine de l'invention concerne le domaine des cathéters pour des interventions sur le coeur qui nécessitent d'effectuer une opération précise tout en visualisant l'opération effectuée en temps réel. Enfin, le domaine de l'invention se rapporte aux cathéters souples ou rigides pouvant être introduits dans le corps humain de manière percutanée, par voies intravasculaires, ou pouvant être directement introduits par ponction d'une cavité cardiaque, telle que l'oreillette ou le ventricule, ou encore par un axe vasculaire tel qu'une veine pulmonaire.

### ETAT DE L'ART

Actuellement, il existe des cathéters pour visualiser des zones du coeur lors d'une opération nécessitant un contrôle visuel de la zone à opérer. Il existe également des cathéters comportant un élément d'intervention pour opérer une zone du coeur. Typiquement, un élément d'intervention permet par exemple de procéder à une pose de clip sur une valve. Il existe des opérations plus complexes par exemple d'annuloplastie visant à renforcer, fixer la taille ou réduire l'anneau d'insertion de la valve mitrale ou tricuspide.

Lors de telles opérations de nombreux instruments sont nécessaires. Il est parfois nécessaire d'introduire différents cathéters pour mener différentes opérations sur un organe du coeur tel qu'une valve.

Les risques relatifs à de telles opérations sont considérablement réduits lorsqu'une visualisation des opérations peut être restituée en temps réel. Un inconvénient des solutions actuelles est que les sondes sont souvent situées à l'extérieur du corps et nécessitent l'enregistrement des images du coeur par l'intermédiaire du thorax. Les images subissent une dégradation inhérente à la structure du corps traversé par les ondes, des organes limitrophes causant des réflexions d'ondes, des artefacts causés par la graisse ou les poumons et des échos parasites des ondes réfléchies. En outre, il est difficile de disposer la sonde correctement vis-à-vis de l'opération à mener et en fonction de l'orientation de l'élément d'intervention.

Lorsqu'une sonde échographique est introduite dans l'organe, ici le coeur ou à sa proximité, un problème d'encombrement se pose. En effet, les zones d'opérations sont confinées dans des espaces de petites dimensions et il est difficile d'acheminer, en plus des instruments médicaux, des sondes permettant de visualiser les opérations conduites sur une partie d'un organe.

Certains dispositifs permettent d'intégrer une sonde, mais ces dernières sont souvent de grandes dimensions et sont difficiles à manipuler en combinaison d'une manipulation d'autres instruments. Ils n'offrent, en général, pas toute la flexibilité nécessaire et imposent une orientation définie du cathéter lors de son introduction qui peut ne pas être compatible avec le mode opératoire.

En effet, les opérations généralement conduites au niveau de la valve atrio-ventriculaire droite ou gauche demandent une grande précision et une certaine manoeuvrabilité offrant une flexibilité au cathéter. Certaines opérations, comme le traitement des fuites, la suture, l'annuloplastie, etc., restent difficiles à mener car le cathéter est difficile à positionner et à stabiliser pour mener à bien une opération sous contrôle visuel.

En ce qui concerne les méthodes pour intervenir sur les valves atrio-ventriculaires droite ou gauche et les modes opératoires associés, de nombreux inconvénients résultent de l'absence d'un cathéter permettant d'obtenir une image précise des opérations menées sur un organe.

Le traitement de référence dans la réparation de la valve atrio-ventriculaire droite ou gauche associe en fait un geste sur la valve elle-même et un geste sur l'anneau valvulaire, appelé annuloplastie. Ce dernier geste consiste à "fixer la taille et à resserrer" l'anneau valvulaire soit par l'usage d'une suture le long de cet anneau, soit par la mise en place d'un anneau prothétique de la taille adéquate qui est directement suturé au contact de l'anneau valvulaire. Le traitement percutané et/ou mini-invasif des pathologies valvulaires mitrales et tricuspides repose essentiellement sur des gestes réalisés sur les feuillets valvulaires, comme par exemple pour la pose d'un ou des Mitraclip ou d'un ou des Neochords.

La limite actuelle des dispositifs d'annuloplastie mitrale percutanés ou mini-invasifs vient de la difficulté à réaliser de manière reproductible, fiable et en sécurité la mise en place de sutures exactement au niveau de l'anneau valvulaire.

Les difficultés liées à la mise en place de telles sutures viennent de la difficulté à visualiser de manière très précise l'anneau valvulaire que ce soit en échographie trans-thoracique ou trans-oesophagienne. En effet, ses rapports anatomiques tant avec le ventricule qu'avec l'oreillette rendent la mise en place des sutures à risque potentiel de perforation atriale ou ventriculaire, d'échec de prise de l'anneau valvulaire dans la suture, c'est-à-dire le risque de fragilisation de la suture, et pour la région commissurale antérieure mitrale de risque de perforation aortique.

Les cathéters typiques dans ce contexte sont décrits dans US 2004/068191 A1 et US 2011/021911 A1.

### RESUME DE L'INVENTION

L'invention vise à pallier les inconvénients précités.

Un objet de l'invention concerne un cathéter selon la revendication 1.

Un avantage est de permettre de disposer d'un élément d'intervention dans une zone qui peut être contrôlée visuellement lors d'une opération. L'élément d'intervention est avantageusement pilotable dans sa rotation par un moyen de pilotage à distance. Le cathéter de l'invention permet donc un gain de précision et une latitude de mouvement d'un élément d'intervention dans une zone visualisable.

Avantageusement, la région de rayonnement latéral du faisceau est sensiblement comprise dans un premier plan. Selon un mode de réalisation, la sonde est une sonde échographique ultrasonore. La sonde échographique ultrasonore permet l'émission et la réception du faisceau ultrasonore. Elle est branchée à l'extérieur sur la console de commande et permet le traitement du signal et la visualisation des images qu'elle génère. La première fenêtre est avantageusement une fenêtre ultrasonique, c'est-à-dire ne filtrant pas les ondes ultrasonores.

Un avantage est de permettre une visualisation dans le plan du faisceau des opérations menées par l'(les) élément(s) d'intervention lui(eux)-même(s) piloté(s) à distance par un moyen de contrôle de sa(leur) position et/ou de son(leur) orientation dans le plan du faisceau. Un tel moyen de contrôle peut comprendre une poignée de contrôle.

La fenêtre latérale est particulièrement adaptée aux opérations sur les valves du coeur et/ ou des anneaux à leur base. Le cathéter est préférentiellement introduit entre l'oreillette et le ventricule et permet une visualisation précise des opérations conduites sur l'anneau et sur la valve.

Un avantage d'un rayonnement plan est la précision accrue de la visualisation. Elle permet d'obtenir par construction un alignement entre la fenêtre de visualisation et les déplacements des éléments d'intervention.

Avantageusement, le cathéter comprend un second dispositif de guidage longitudinal permettant de diriger au moins un bras d'une sonde amovible à l'intérieur du corps creux du cathéter et permettant de positionner la sonde en regard de la première fenêtre.

Selon un mode de réalisation, la sonde est capable de se déplacer longitudinalement dans le corps creux et est associée à un dispositif de maintien permettant d'assurer la stabilité de la sonde lorsqu'elle est positionnée en regard de la première fenêtre.

Un avantage est que le cathéter peut être un consommable interchangeable.

Selon un autre mode de réalisation, la sonde est fixée au corps creux.

Avantageusement, un élément déformable est mobile dans le premier plan P1 au moyen d'un dispositif de guidage, l'élément déformable étant adapté à coopérer avec un élément d'intervention à son extrémité distale.

Avantageusement, l'élément d'intervention est fixé et guidé à l'extrémité distale de l'élément déformable par l'intermédiaire d'un bras distal.

Avantageusement, l'élément déformable comporte une gaine, ledit élément d'intervention étant introduit à l'intérieur de la gaine. L'élément d'intervention et/ou l'extrémité de l'élément déformable sont guidés dans le plan du faisceau.

Avantageusement, au moins un dispositif de guidage angulaire est associé à au moins un dispositif de guidage longitudinal de sorte à engager un mouvement de rotation pour piloter l'angle d'intervention, le mouvement de rotation étant engagé par un moyen de pilotage à distance.

Avantageusement, le mouvement de rotation entraîne la rotation d'un bras mobile distal du dispositif de guidage angulaire guidant soit l'extrémité distale d'un élément déformable, soit un élément d'intervention, soit l'ensemble formé de l'extrémité distale d'un élément déformable et d'un élément d'intervention.

Avantageusement, les moyens de pilotage à distance permettent la conversion d'un mouvement de translation d'un bras du dispositif de guidage angulaire en un mouvement de rotation d'un bras mobile distal.

Avantageusement, le mouvement de translation d'un bras du dispositif de guidage angulaire est opéré au moyen d'un dispositif de guidage longitudinal.

Avantageusement, au moins un moyen de pilotage à distance comprend une poignée ou une molette dont la rotation permet de piloter l'ouverture de l'angle d'intervention de l'élément d'intervention.

Avantageusement, au moins un dispositif de guidage longitudinal comprend soit une lumière longitudinale, soit des arceaux, soit un rail, le rail pouvant être à l'intérieur ou à l'extérieur du cathéter et solidaire de ce dernier.

Avantageusement, au moins un dispositif de guidage longitudinal permet le guidage longitudinal de tout ou d'une partie d'un dispositif de guidage angulaire et/ou d'un élément déformable et/ou d'un élément d'intervention.

Avantageusement, au moins un dispositif de guidage longitudinal comprend une lumière longitudinale s'étendant à l'intérieur du cathéter, ledit dispositif de guidage longitudinal étant associé à son extrémité distale à un dispositif de guidage angulaire et étant maintenu dans un plan P1 comprenant l'axe du cathéter.

Avantageusement, l'élément déformable est souple et est introduit dans la lumière longitudinale par l'extrémité proximale du cathéter, le corps creux du cathéter comprenant une ouverture latérale dont la plus grande dimension est comprise dans le même plan que la plus grande dimension de la première fenêtre, ladite ouverture latérale étant adaptée pour permettre les mouvements de rotation d'un dispositif de guidage angulaire et pour le passage d'au moins un élément d'intervention à l'extérieur du cathéter.

Avantageusement, un élément déformable est guidé par un dispositif de guidage angulaire comprenant une tige poussoir translatant sur un dispositif de guidage longitudinal solidaire du cathéter, la translation de la tige poussoir actionnant une liaison pivot du dispositif de guidage angulaire, la tige poussoir étant actionnée par les moyens de pilotage à distance, la liaison pivot engageant une rotation d'un bras mobile distal supportant un élément d'intervention.

Avantageusement, le dispositif de guidage longitudinal comprend un rail solidaire du corps du cathéter s'étendant longitudinalement dans le premier plan P1 et permettant la translation de la tige poussoir du dispositif de guidage angulaire.

Avantageusement, au moins un dispositif de guidage angulaire comporte un fil permettant de maintenir un bras mobile distal en position fermée, une liaison pivot du dispositif de guidage angulaire étant couplée à un élément de rappel exerçant une force de rappel tendant à ouvrir l'élément d'intervention dans le plan du faisceau, une action sur le fil permettant de faire pivoter l'élément d'intervention dans le plan du faisceau.

Avantageusement, au moins un dispositif de guidage angulaire comporte un fil permettant de maintenir un bras mobile distal en position ouverte, une liaison pivot du dispositif de guidage angulaire étant couplée à un élément de rappel exerçant une force de rappel tendant à fermer l'élément d'intervention le long du cathéter, une action sur le fil permettant de faire pivoter l'élément d'intervention dans le plan du faisceau.

Avantageusement, le cathéter comprend un premier élément déformable et un second élément déformable associés respectivement à un premier et à un second éléments d'intervention, deux dispositifs de guidage angulaires associés à deux dispositifs de guidage longitudinaux, les deux éléments d'intervention débouchant respectivement en amont et en aval de la première fenêtre de sorte à ce qu'un premier élément d'intervention et respectivement un second élément d'intervention puissent évoluer dans le plan du faisceau simultanément ou successivement.

Un autre objet de l'invention concerne un cathéter comprenant un corps creux s'étendant longitudinalement et comportant au moins trois guides alignés dans un premier plan P1 colinéaires à l'axe du cathéter, un premier guide étant apte à guider longitudinalement une sonde échographique amovible et un second et un troisième guides étant aptes à guider au moins longitudinalement chacun un dispositif de guidage angulaire et/ou un élément déformable et/ou un élément d'intervention, le premier guide débouchant sur une première fenêtre latérale du corps du cathéter, le second guide débouchant en amont de la fenêtre et le troisième guide débouchant en aval de la fenêtre, le cathéter comprenant en outre au moins un dispositif de guidage angulaire orientable associé à un des second ou troisième guides et dont les mouvements de rotation sont compris dans le premier plan P1.

Avantageusement, le cathéter comprend au moins une ouverture latérale dont la plus grande dimension est comprise dans le même plan que la plus grande dimension de la première fenêtre , le second ou le troisième guide débouchant sur l'ouverture latérale du cathéter.

Avantageusement, au moins un des second et/ou troisième guide(s) comprend un rail longitudinal solidaire du corps du cathéter.

Avantageusement, l'un des second et/ou troisième guide(s) comprend une lumière tubulaire s'étendant longitudinalement à l'intérieur du corps creux du cathéter.

Avantageusement, le cathéter comprend au moins un élément déformable coopérant avec au moins un dispositif de guidage de guidage angulaire.

Avantageusement selon les différents objets de l'invention, le cathéter comporte un canal d'irrigation à l'intérieur du cathéter dont le débit d'un fluide d'irrigation peut être contrôlé au moyen d'un dispositif de contrôle du fluide.

Avantageusement selon les différents objets de l'invention, le cathéter est apte à coopérer avec un introducteur comportant une ventouse à son extrémité distale pour adhérer par aspiration à un organe à perforer, ledit introducteur comportant un canal opérateur permettant le passage dudit cathéter à l'intérieur dudit organe.

Avantageusement, la ventouse comprend un canal opérateur central ne communiquant pas avec la partie aspirative de la ventouse.

Avantageusement, la ventouse est associée à un dispositif permettant de contrôler par serrage la mobilisation longitudinale du cathéter à travers la ventouse, ainsi que les mouvements latéraux du cathéter au travers de la ventouse.

### BREVE DESCRIPTION DES FIGURES

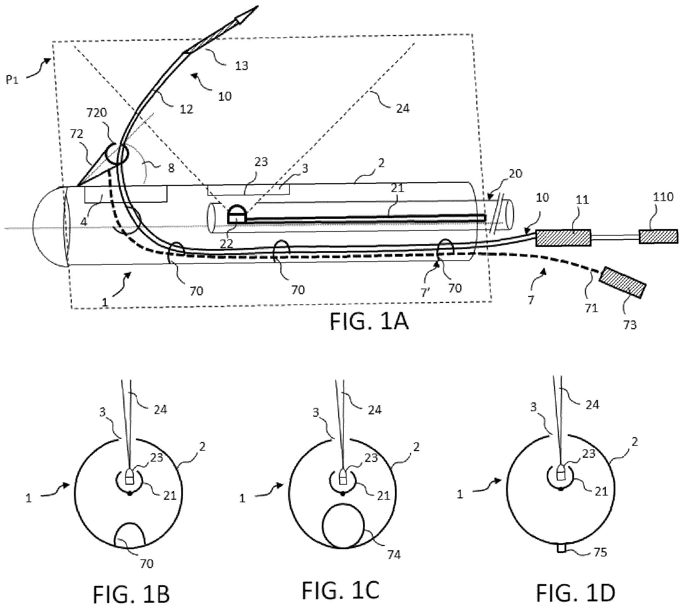

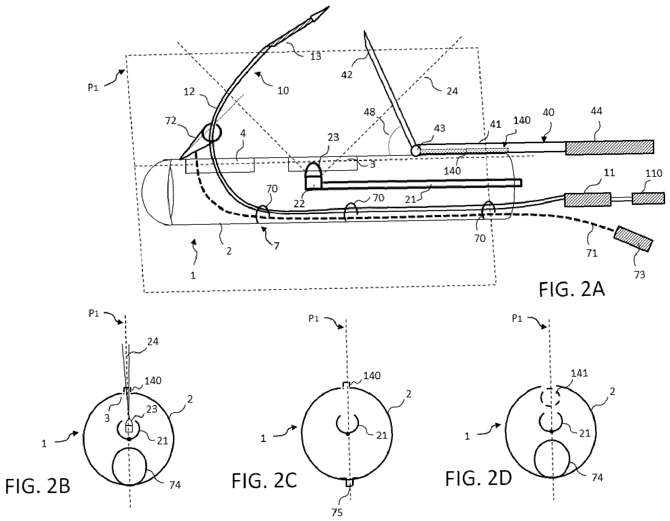

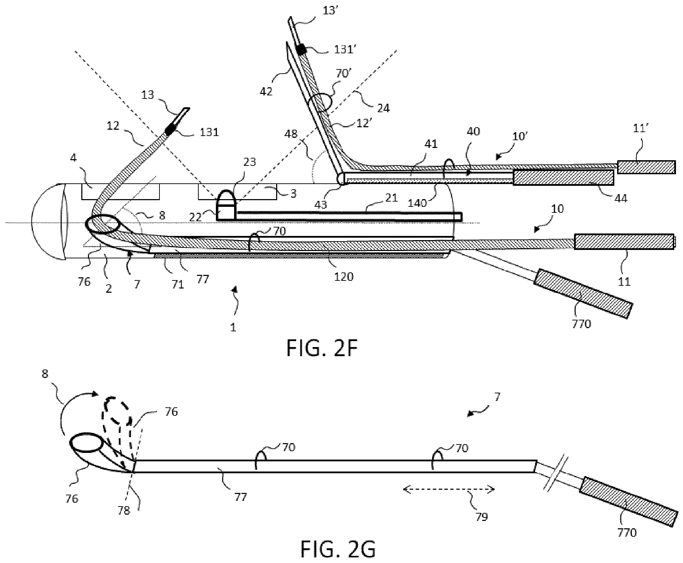

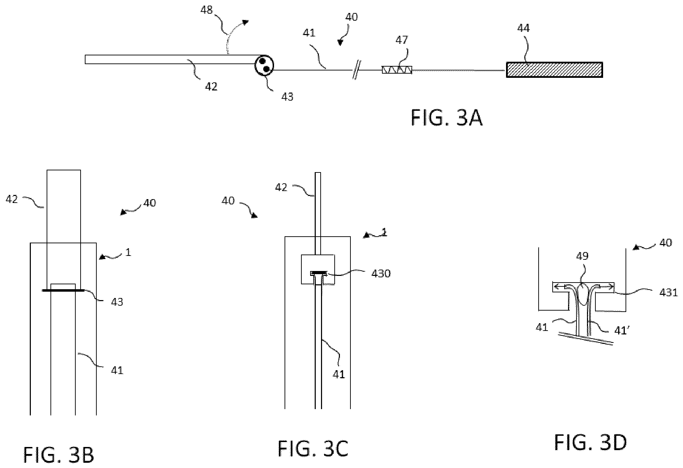

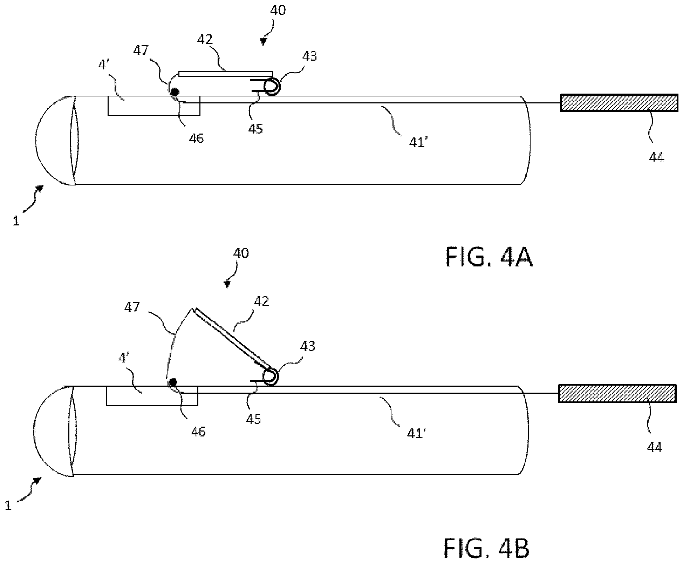

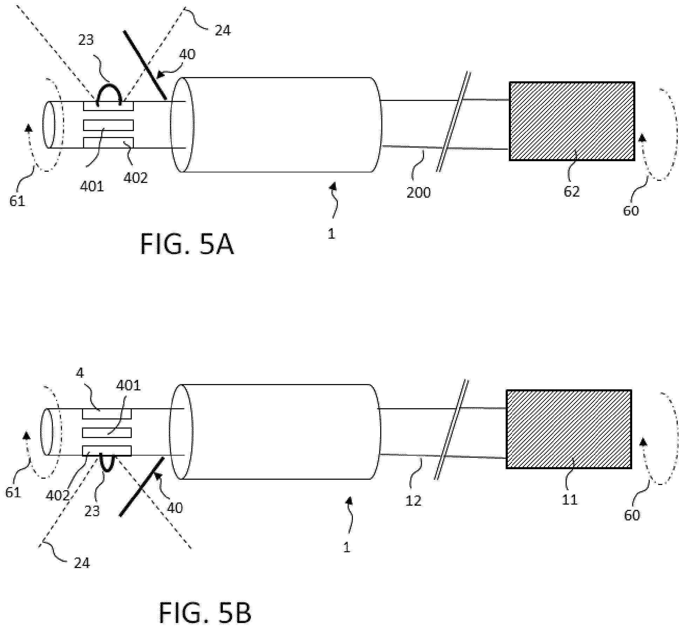

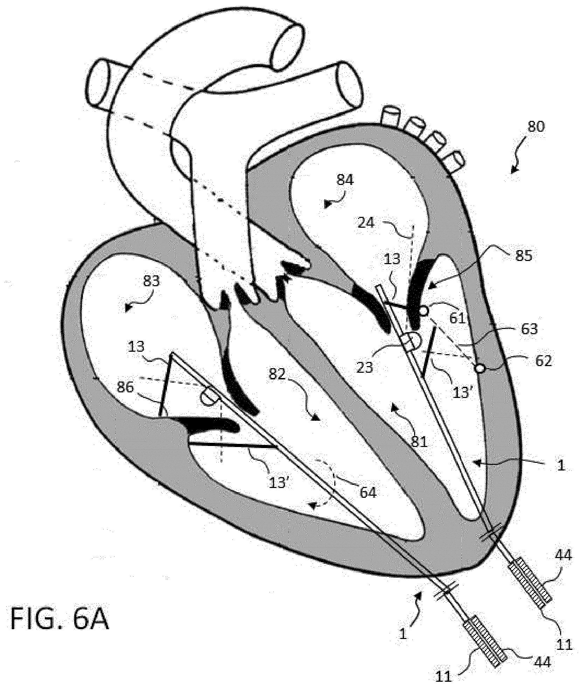

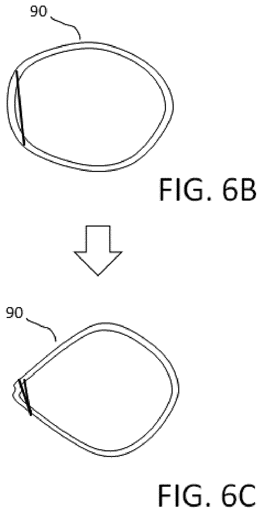

D'autres caractéristiques et avantages de l'invention ressortiront à la lecture de la description détaillée qui suit, en référence aux figures annexées, qui illustrent :
▪ Figure 1A: une vue longitudinale d'un cathéter selon un premier mode de réalisation de l'invention en vue de profil comprenant un élément déformable et les dispositifs de guidage longitudinal et angulaire associés à l'élément déformable ;
▪ Figures 1B à 1D : des vues de coupes de la section d'un cathéter de l'invention selon différentes variantes de réalisation des moyens de guidage longitudinaux ;
▪ Figures 2A, 2F: un cathéter selon un second mode de réalisation de l'invention comprenant deux éléments déformables et les dispositifs de guidage longitudinaux et angulaires associés;
▪ Figure 2G : un mode de réalisation d'un dispositif de guidage longitudinal ;
▪ Figures 2B à 2E : des vues de coupes de la section d'un cathéter de l'invention selon différentes variantes de réalisation de dispositifs de guidage longitudinaux ;
▪ Figures 3A, 3B, 3C, 3D : différentes variantes d'un dispositif de guidage longitudinal associé à un dispositif de guidage angulaire d'un cathéter de l'invention permettant d'actionner un élément d'intervention ;
▪ Figures 4A, 4B : un cathéter comprenant un élément d'intervention piloté à partir d'un élément déformable et d'un dispositif de guidage angulaire ;
▪ Figures 5A, 5B : un cathéter de l'invention comprenant une sonde échographique orientable selon un mode de réalisation de l'invention ;
▪ Figure 6A : deux représentations d'introduction d'un cathéter selon l'invention par le ventricule gauche ou droit pour une opération sur une valve mitrale ou tricuspide, respectivement d'une intervention sur l'anneau de la valve atrio-ventriculaire droite et d'une intervention sur le feuillet de la valve atrio-ventriculaire gauche ;
▪ Figure 6B et 6C : le résultat d'une intervention sur un anneau d'une valve après l'exécution d'une méthode de l'invention.

### DESCRIPTION

Dans la suite de la description, on appelle « cathéter » un dispositif médical comprenant un tube ayant une largeur et une souplesse variables et fabriqué en différentes matières selon les modèles ou les usages pour lesquels ils sont destinés.

Le cathéter est destiné à être inséré :
- soit dans une cavité du corps, la cavité pouvant être naturelle ou réalisée par un acte de chirurgie ;
- soit dans un vaisseau sanguin.

Le cathéter peut soit être destiné au drainage ou à l'infusion de liquides, ou encore permettre un accès à un ou plusieurs dispositifs médicaux ou permettre l'introduction de dispositifs d'imagerie.

Un introducteur souple peut donc être entendu dans la présente description comme un cathéter permettant l'introduction d'autres instruments médicaux.

On entend par « ouverture latérale » du cathéter une ouverture au sens d'une fenêtre permettant le passage d'ondes d'une sonde d'imagerie. Dans le cas d'une utilisation d'une sonde ultrasonore, l'ouverture est une fenêtre ultrasonique. L'ouverture peut être une ouverture mécanique réalisée dans le corps du cathéter. L'ouverture peut être en outre recouverte d'un cache, amovible ou fixé, comme un élément vitreux, ne filtrant pas les ondes ultrasonores de la sonde.

On entend par « lumière longitudinale » un guide tubulaire ou de forme équivalente disposé à l'intérieur du cathéter et s'étendant longitudinalement dans son corps creux permettant le passage d'un élément déformable ou d'une sonde ou d'un élément d'intervention.

La figure 1A représente un mode de réalisation de l'invention. Un cathéter 1 comprend un corps creux 2, par exemple tubulaire, dans lequel une sonde échographique 23 est introduite par une lumière proximale du cathéter et peut se déplacer longitudinalement dans le corps creux. Dans un mode de réalisation, le corps 2 est une gaine souple par exemple pour des applications percutanées. Selon un autre mode, le corps est rigide, par exemple pour des applications chirurgicales telles que l'introduction du cathéter 1 directement dans un des ventricules du coeur ou dans une des oreillettes droite ou gauche lors d'une intervention chirurgicale.

### Sonde

Dans un mode de réalisation, la sonde 23 comprend un dispositif de guidage longitudinal 20 guidant un bras 21 qui peut être souple ou rigide selon la combinaison envisagée avec le corps 2 du cathéter 1. La sonde 23 comprend un transducteur permettant d'émettre un faisceau d'ondes dans un cône 24 et de recevoir les échos des ondes émises et réfléchies ainsi que de transférer à une console de commande et de visualisation externe les données permettant la constitution de l'image échographique. Le cône est préférentiellement sensiblement plan. Une fenêtre ultrasonique latérale, représentée sous forme d'ouverture 3 latérale, conçue dans le corps 2 du cathéter 1 permet l'émission d'un rayonnement de l'émetteur 23 à l'extérieur du cathéter 1 vers un organe à éclairer.

La tête 22 de la sonde est préférentiellement orientée de sorte à émettre un faisceau parallèle à l'axe longitudinal du cathéter 1. Préférentiellement, le plan comprenant le faisceau comprend l'axe longitudinal du cathéter 1.

L'analyse des échos permet d'obtenir une imagerie en temps réel et précise. L'échographie étant localisée, les images obtenues permettent d'obtenir une représentation précise de certains organes du coeur tels qu'une valve mitrale ou tricuspide, son anneau ainsi que les structures anatomiques environnantes.

Selon un mode de réalisation, la sonde est une sonde échographique comprenant des cellules à ultrasons. Le faisceau ainsi généré est un faisceau ultrasonore. Le nombre et la disposition des cellules de la sonde peuvent être configurés selon l'application envisagée. Par exemple, la sonde peut comprendre quelques cellules ou 16, 32, 64 ou plus de cellules encore. Les cellules sont agencées longitudinalement ou sous forme de matrice.

Selon un mode de réalisation, l'émetteur 23 peut être maintenu dans une position fixe à l'intérieur du corps 2 du cathéter 1 de sorte à diriger le faisceau vers l'extérieur du cathéter au travers la fenêtre 3. Pour cela, des moyens de maintien peuvent être utilisés comme par exemple une butée disposée à l'intérieur du corps 2 ou un serrage par vis le long du corps 2 du cathéter 1.

Lorsque la sonde 23 est directement fixée à l'intérieur du cathéter 1 sans être amovible, l'émetteur est solidaire du corps 2 du cathéter 1. Le cathéter de l'invention peut être par exemple un cathéter d'imagerie comprenant une sonde ultrasonore d'imagerie solidaire.

Selon une variante de réalisation qui peut être combinée avec les moyens de maintien, un dispositif de rotation de l'ensemble de l'extrémité du cathéter 1 suivant son axe longitudinal peut être installé à l'intérieur du cathéter 1. Ce dispositif de rotation permet de contrôler l'orientation de la tête 22 de la sonde échographique 23. L'ensemble de l'extrémité du cathéter 1 comprend dans ce mode de réalisation : la sonde échographique et au moins une des zones permettant le passage d'un élément déformable et/ou d'un élément d'intervention. Un tel dispositif de rotation suivant l'axe longitudinal est décrit par la suite au moyen du contrôle de deux fils de guidage.

### Faisceau

Selon un mode de réalisation préféré de l'invention, le cône 24 d'émission de la sonde 23 est compris sensiblement dans un plan P1. Dans ce dernier cas les cellules de la sonde sont préférentiellement agencées longitudinalement. La limitation de la zone d'imagerie à une zone sensiblement comprise dans un plan permet d'obtenir une image précise dans le faisceau. Par ailleurs, si un élément d'intervention du cathéter est guidé dans le plan du faisceau, la connaissance de sa position radiale est connue par construction. En outre, les images obtenues permettent de suivre précisément les mouvements d'un tel élément d'intervention dans le plan à l'intérieur de l'organe.

Le plan P1 de la figure 1A représente le plan comprenant le faisceau 24. Les figures 1B, 1C et 1D représentent une section du cathéter 1, la coupe étant réalisée au niveau de la fenêtre 3. Ces figures représentent également le plan du faisceau 24 qui comprend une légère ouverture.

Selon un mode de réalisation de l'invention, le faisceau 24 peut avoir un angle d'ouverture en largeur perpendiculaire à l'axe longitudinal du cathéter 1 de quelques degrés. On appelle l' « angle d'ouverture en largeur » dans la présente description plus simplement par un « angle d'ouverture ». Cette dernière ouverture reste considérée sensiblement proche d'un faisceau compris dans un plan lorsque l'ouverture est inférieure à 15°. Le faisceau est dans ce cas suffisamment directif pour permettre la génération d'une imagerie très précise.

Outre son ouverture latérale, le faisceau peut avoir des angles d'ouverture longitudinaux adaptés à étendre angulairement de part et d'autre le faisceau longitudinalement selon l'axe du cathéter 1. Par exemple, un angle de 45° avec l'axe longitudinal du cathéter 1 permet d'étendre la couverture du faisceau au-delà des extrémités distale et proximale de la fenêtre ultrasonique. L'ouverture longitudinale peut être évasée par exemple si la sonde est bombée. L'ouverture longitudinale du faisceau peut être identique de part et d'autre de la fenêtre ultrasonique, c'est-à-dire sur le bord distal et le bord proximal de la fenêtre ultrasonique.

On appelle un « plan latéral » au cathéter 1, un plan dont les dimensions s'étendent longitudinalement au cathéter 1. Le plan latéral est parallèle à l'axe du cathéter 1. Il comprend préférentiellement l'axe du cathéter 1, mais selon un autre mode de réalisation, il pourrait également être légèrement oblique et/ou orienté vis-à-vis de l'axe d'un rayon du cercle formé par une coupe transversale du cathéter 1.

Dans une variante de réalisation, on configure la sonde pour obtenir une ouverture inférieure à 10°. Cette ouverture est donc également considérée sensiblement proche d'un plan. Le faisceau plan 24 peut être obtenu :
- soit par l'émission d'un faisceau d'ondes dans un plan au moyen d'un émetteur adapté ;
- soit par la limitation de la fenêtre 3 en forme de fente qui permet le passage des ondes émises selon un plan comprenant l'axe longitudinal du cathéter 1.

Le faisceau 24 plan peut comprendre une certaine largeur tolérée de quelques millimètres étant donné qu'à une certaine distance de quelques centimètres, le faisceau s'élargit du fait de son ouverture.

L'avantage de la génération d'un faisceau plan est qu'il permet de réduire le nombre de cellules nécessaires dans la sonde. Par exemple, chaque émetteur/récepteur peut être une céramique piézoélectrique. Les cellules utilisées peuvent être agencées sous forme de matrice de cellules réduite par exemple de forme longitudinale. Cette option permet de réduire l'encombrement d'un émetteur et permet une intégration d'une sonde dans un cathéter 1 au corps 2 souple. En effet, la génération d'une image locale selon une dimension principale, ici longitudinale, permet de réduire considérablement le nombre de cellules nécessaire tout en conservant une image de haute définition. Il s'agit là d'un avantage considérable pour les sondes embarquées notamment dans des cathéters souples destinés à être introduits par voie percutanée ou par voie intraveineuse.

Selon un mode de réalisation de l'invention, les éléments générant le faisceau ultrasonore sont disposés en matrice permettant l'obtention d'une imagerie dite en 3 dimensions dite 3D. Une ou des cellules piézoélectriques peuvent être utilisées à ces fins.

### Eléments déformables

Selon l'invention, un premier élément déformable 10, qui forme un bras souple, permet de guider un élément d'intervention 13. Un moyen de pilotage 11 des mouvements de l'élément déformable permet par exemple d'opérer des mouvements à distance de l'élément d'intervention 13 à l'extrémité distale du cathéter. Le dispositif déformable peut comprendre dans un mode de réalisation un bras distal 12 adapté à coopérer avec l'élément d'intervention 13. L'extrémité distale de l'élément déformable 10 est appelée plus généralement dans la suite de l'invention « bras distal » 12. Un dispositif de guidage angulaire 7 permet de contrôler l'angle d'intervention 8 et de guider l'élément d'intervention 13 ou le bras distal 12 de l'élément déformable 10 dans le plan du faisceau 24.

Selon un mode de réalisation de l'invention, un élément déformable 10 est une gaine de type cathéter d'angiographie. Selon un autre mode de réalisation de l'invention, un élément déformable est un rail sur lequel glissera l'élément d'intervention.

Le bras déformable distal 12 de l'élément déformable 10 permet de guider l'élément d'intervention 13 dans le plan du faisceau 24. En outre, l'élément d'intervention 13 peut s'insérer longitudinalement dans l'élément déformable 10 à partir de sa région proximale et sortir de manière plus ou moins complète à son extrémité distale. L'élément d'intervention 13 peut être introduit dans l'élément déformable et guidé par une poignée 110.

Selon un mode de réalisation, le premier élément déformable 10 est introduit dans la cavité creuse du corps 2 du cathéter 1 disposant d'une lumière à son extrémité proximale. Le bras distal 12 peut être guidé dans son orientation angulaire 8 au moyen, par exemple, d'un dispositif de guidage angulaire 7 dont une partie mobile distale 72 comprend dans cet exemple un anneau 720. La « partie mobile distale » 72 est appelée dans la suite de la description « bras mobile distal » 72 du dispositif de guidage angulaire 7. La même appellation est déterminée pour le bras mobile distal 42 du dispositif de guidage angulaire 40.

Le dispositif de guidage angulaire 7 est associé à un dispositif de guidage longitudinal 7' ici représenté sous forme d'anneaux dans l'exemple de la figure 1.

La partie distale 72 du dispositif de guidage angulaire est contrôlable par un bras principal 71 et une poignée 73. A titre d'exemple le contrôle peut être effectué en actionnant une liaison pivot ou en modifiant la longueur d'un fil 41' à l'aide de la poignée 73. L'angle 8, qui définit sensiblement l'angle d'intervention, c'est-à-dire l'angle d'attaque de l'élément d'intervention 13 dans le faisceau 24, est donc ajustable.

### a-Elément d'intervention

L'élément d'intervention 13 permet d'intervenir sur une partie d'un organe afin de le maintenir, d'y insérer ou suturer un dispositif implantable ou de le coudre par exemple. L'élément d'intervention 13 évolue dans une zone pouvant s'étendre de quelques millimètres de la surface du cathéter 1 à quelques centimètres, voire jusqu'à une dizaine de centimètres.

L'élément d'intervention 13 peut être une aiguille ou une aiguille de radiofréquence, une sonde d'enregistrement électrique, une pince de type « pince à biopsie » ou une pince à préhension par exemple. En outre, l'élément d'intervention peut également être un élément de ponction, une sonde d'angiographie, une sonde à ballonnet, un guide, une sonde laser, un lasso permettant de récupérer un guide ou un fil, un dispositif permettant la fixation d'un clip d'une suture ou d'une agrafe ou encore un élément délivrant de la chaleur ou abaissant la température. Un élément délivrant de la chaleur peut être par exemple une sonde radiofréquence et un élément abaissant la température peut être par exemple une sonde cryode. Les éléments d'intervention peuvent permettre la fixation de points, d'agrafes, de clips. En outre, ils peuvent permettre d'effectuer des sutures intravasculaires ou de fixer un dispositif implantable tel qu'un anneau prothétique ou un substitut valvulaire.

Selon un mode de réalisation, l'élément d'intervention 13 utilise un élément déformable 10 et/ou un dispositif de guidage angulaire 72 pour être mobilisé dans le faisceau ultrasonique 24.

Selon un autre mode de réalisation, l'élément d'intervention 13 est mobilisable de par ses propriétés propres et n'utilise pas l'élément déformable et/ou le dispositif de guidage angulaire.

Une aiguille permet de perforer l'anneau d'une valve. Cette opération permet, par exemple, d'introduire un fil dans l'ouverture effectuée. Un avantage de l'invention est de permettre un gain en précision lors de la manipulation d'une telle aiguille d'une dimension de quelques dixièmes de millimètres en pointe.

Une autre possibilité est de disposer d'un élément ressort entre deux ouvertures effectuées sur un anneau à partir d'une aiguille de ponction. Cette opération permet, par exemple, de resserrer deux points de la circonférence d'un anneau d'une valve et donc de diminuer le périmètre de l'anneau.

### c-Dispositif de guidage

Deux types de dispositifs de guidage sont décrits et associés dans cette invention.
∘ un dispositif de guidage longitudinal ;
∘ un dispositif de guidage angulaire ;

Selon les configurations les deux dispositifs de guidage longitudinal et angulaire coopèrent en ayant des éléments communs ou pas de sorte à permettent des déplacements de l'élément d'intervention dans le plan du faisceau 24.

Le cathéter 1 peut comprendre, selon les modes de réalisation, un ou plusieurs dispositifs de guidage longitudinal(aux) suivant l'axe principal du cathéter 1.

Le ou les dispositifs de guidage longitudinal peuvent être sous la forme, combinée ou non, d'anneaux, de rail, de gaine ou de lumière, situés dans la lumière du cathéter 1. Avantageusement, les mêmes types de guidage longitudinaux peuvent être situés à l'extérieur du corps du cathéter 1.

Concernant le dispositif de guidage longitudinal, selon les modes de réalisation de l'invention, il permet le guidage d'au moins l'un des éléments suivants:
- la sonde échographique ;
- et/ou d'un dispositif de guidage angulaire lorsque ce dernier comporte un bras d'actionnement d'une liaison pivot telle que l'élément 72;
- et/ou d'un élément déformable et/ou d'un élément d'intervention le long du cathéter 1 ;
- et/ou d'un élément déformable et/ou d'un élément d'intervention le long du bras principal et/ou du bras distal mobile du dispositif de guidage angulaire.

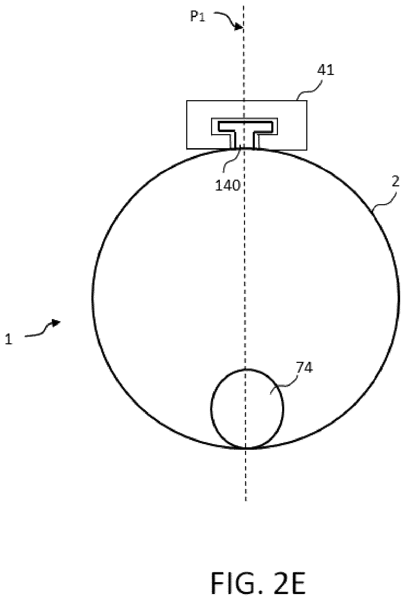

Le ou les dispositifs de guidage longitudinal(aux) peut(vent) être implémenté(s) le long du cathéter 1 selon différentes variantes de réalisation représentées de manière non limitative aux figures 1A, 1B, 1C, 1D, 2A, 2B, 2C, 2D, 2 E et 2F.

Concernant le ou les dispositifs de guidage angulaire, ils permettent la mobilisation si nécessaire du bras distal souple 12 de l'élément déformable 10 et/ou si nécessaire de l'élément d'intervention 13 dans le plan du faisceau ultrasonique 24.

Selon un mode de réalisation, le dispositif de guidage angulaire 7 comprend :
- un bras mobile distal 72, qui dirige la rotation angulaire 8 de l'élément déformable 10 ou de son bras distal 12 et/ou de l'élément d'intervention 13 ;
- un bras principal 71 permettant de piloter le bras mobile distal 72 ;
- un moyen de pilotage à distance 73 permettant d'agir sur le bras principal 71.

Il peut être également défini suivant deux autres modalités :
- une première modalité est représentée à la figure 3A par une tige poussoir 41 dont la translation actionne une liaison pivot 43, la tige poussoir étant actionnée par les moyens de pilotage à distance 44, la liaison pivot 43 engageant une rotation du bras mobile distal 42 servant de support et permettant la mobilisation de l'élément déformable (non représenté) et/ou de l'élément d'intervention (non représenté) dans une zone du plan du faisceau 24.
   Dans cette première modalité, la tige poussoir 41 peut être entendue aussi bien comme un élément d'un dispositif de guidage longitudinal étant donné le mouvement de translation et coopérant avec le dispositif de guidage angulaire 43 et 42 ou comme un élément d'un dispositif de guidage angulaire étant donné que c'est un élément actionnant la liaison pivot 43. Il peut être combiné à un autre dispositif de guidage longitudinal permettant d'acheminer un élément d'intervention 13 utilisant le bras 42 comme support.
   Le bras 42 formant un support à un élément d'intervention peut également être directement un élément d'intervention.
- Une seconde modalité est représentée aux figures 4A et 4B dans lesquelles un bras mobile distal 42 est combiné avec des éléments 43, 45 d'un dispositif de guidage angulaire 40 pour le contrôle angulaire dudit bras mobile distal 42. Le dispositif de guidage angulaire 40 comprend un élément de rappel 43 exerçant une force de rappel et un fil 41' comportant une portion 47 extérieure au cathéter 1.
   Quatre variantes sont décrites aux figures 4A, 4B.
   De la même manière, le bras mobile 42 peut être entendu comme un support pour un élément déformable 10' ou un support pour un élément d'intervention 13' mais aussi comme l'élément d'intervention lui-même selon le mode de réalisation envisagé.
   Le fil 41' peut être entendu comme un dispositif de guidage longitudinal dans le sens ou il opère une translation selon l'axe longitudinal du cathéter 1 ou comme un élément du dispositif de guidage angulaire dans la mesure où il actionne la rotation en libérant le bras 42. Il peut être associé à un autre dispositif de guidage longitudinal tel qu'une lumière permettant d'acheminer un élément d'intervention utilisant le bras 42 comme support.

Dans la variante représentée aux figures 1A et 1B, un dispositif de guidage longitudinal 70 permet d'acheminer l'élément d'intervention 13 en dehors du cathéter 1 au travers d'une ouverture latérale 4. Selon une autre variante, l'ouverture pourrait être réalisée à l'extrémité distale du cathéter à son extrémité. Cette dernière variante nécessiterait un élément de guidage supplémentaire à l'extrémité du cathéter 1 pour ramener l'élément d'intervention dans le plan du faisceau 24.

Dans le cas des figures 1B à 1D, le dispositif de guidage longitudinal 7 comprend des moyens de guidage longitudinaux 70, 74, 75 à l'intérieur ou à l'extérieur du corps 2 du cathéter 1 qui peuvent être des anneaux 70 ou encore un tube ou une gaine 74 ou un rail extérieur 75 situé sur la surface extérieur du cathéter 1. De manière identique et non représentée, un rail intérieur dans le corps 2 du cathéter 1 pourrait être réalisé.

La figure 1B correspond au cas représenté à la figure 1A dans lequel les moyens de guidage longitudinaux 70 sont des anneaux intérieurs dans lesquels l'élément déformable 10 peut se déplacer tout en étant guidé. Les anneaux 70 permettent d'acheminer l'élément déformable 10 et l'élément d'intervention 13 vers l'ouverture 4 ou une ouverture à l'extrémité distale. Le dispositif de guidage angulaire comprenant le bras mobile distal 72 permet d'orienter :
- soit le bras distal 12 de l'élément déformable 10 ;
- soit directement l'élément d'intervention 13 ;
- soit naturellement l'élément déformable 10 et/ou son bras distal 12 et l'élément d'intervention 13 dans le plan du faisceau 24.

On note dans les figures 1B, 1C et 1D, qu'un bras d'un dispositif de guidage longitudinal 21 permet de guider les déplacements d'une sonde 23.

La figure 1C représente le cas d'un tube 74 permettant d'acheminer au moins vers une ouverture latérale 4 ou une ouverture agencée à l'extrémité distale du cathéter 1 :
- un dispositif de guidage angulaire comportant un bras d'actionnement d'une liaison pivot et/ou ;
- un élément déformable 10 et/ou ;
- un élément d'intervention 13.

La figure 1D représente le cas d'un rail extérieur 75 permettant d'acheminer au moins :
- un dispositif de guidage angulaire permettant d'actionner une liaison pivot ou ;
- un bras 10 d'un élément déformable ou ;
- un élément d'intervention 13 vers l'extrémité distale du cathéter 1.

Lorsque l'axe du rail 75 est confondu avec l'axe de la longueur principale de la fenêtre 3 le long de la surface du cathéter 1, alors l'élément d'intervention 13 et l'élément déformable 10 sont en amont de la fenêtre 3, c'est-à-dire sur la partie proximale du cathéter 1 vis-à-vis de la position de la fenêtre 3. Cela évite de faire passer le rail 75 sur la fenêtre et de gêner l'émission et la réception des ondes ultrasonores.

Lorsque l'axe du rail 75 n'est pas confondu avec l'axe de la longueur principale de la fenêtre 3 le long de la surface du cathéter 1, alors le rail 75 est agencé diamétralement opposé à la fenêtre 3 sur la surface extérieure opposée du cathéter 1 (cas de la figure 1D). Ceci impose d'acheminer l'élément déformable 10 jusqu'à l'extrémité distale du cathéter 1 et de faire passer l'élément d'intervention 13 autour de l'extrémité du cathéter 1 pour revenir dans le plan du faisceau 24. Dans ce cas, un dispositif de guidage angulaire 7 dont notamment le bras mobile distal 72 formant un moyen de contrôle de l'angle d'intervention 8 permet de maintenir l'élément d'intervention 13 dans le plan du faisceau 24.

En conclusion, lorsqu'un rail est utilisé, il peut être agencé soit dans l'axe de la fenêtre 3, soit dans un axe parallèle à l'axe principal de la fenêtre et dans le plan du faisceau. Il est donc nécessairement diamétralement opposé à l'axe principal de la fenêtre 3.

En outre, le dispositif de guidage angulaire 7 comprend des moyens de contrôle de l'angle d'introduction de l'élément d'intervention 13 dans le plan du faisceau 24 par rapport à l'axe du cathéter 1. Nous nommons ci-après le « bras mobile distal » un élément pouvant être entendu plus généralement comme un moyen de contrôle de l'angle d'intervention 8.

Dans l'exemple de la figure 1A, le bras mobile distal 72 du dispositif de guidage angulaire 7 est fixé sur le cathéter 1 en regard de la fenêtre d'intervention 4 permettant le passage de l'élément déformable 10 ou de l'élément d'intervention 13. Les moyens de guidage angulaire 7 comprenant un moyen de pilotage à distance 73 pour piloter les mouvements des moyens de contrôle de l'angle 8 du bras mobile distal 72. Il peut s'agir par exemple d'une poignée 73. Selon d'autre variantes de réalisation le moyen de pilotage à distance 73 peut être une molette fixée au cathéter 1 sur sa partie proximale. Pour assurer la liaison entre le moyen de pilotage 73 et les moyens de contrôle 72 de l'angle d'intervention 8, le dispositif de guidage peut comprendre une tige 71 souple ou rigide permettant de contrôler l'angle 8. Un autre mode de réalisation pourrait être la mise en place de fils 71 permettant de régler l'angle de l'élément 72.

Dans un mode de réalisation représenté à la figure 1A, le bras mobile distal 72 permettant de définir l'angle d'intervention 8 est fixé sur le corps 2 du cathéter 1 de sorte à faciliter le guidage de l'élément déformable 10 et de l'élément d'intervention 13 à l'extérieur du cathéter 1 selon une direction donnée. L'élément d'intervention 13 est dirigé dans une zone comprise sensiblement dans le même plan que le plan du faisceau 24. Le bras mobile distal 72 assure la colinéarité du plan 24 et des mouvements de l'élément d'intervention 13. La zone d'intervention est donc dans un plan latéral au cathéter 1 ce qui permet une maîtrise de l'élément d'intervention 13. Le moyen de contrôle 72 de l'angle d'intervention 8 est orientable pour permettre d'orienter l'élément d'intervention 13 dans le plan du faisceau 24. L'extrémité orientable appelée bras mobile distal 72 peut comprendre un anneau 720 dans lequel le bras distal 12 du premier élément déformable 10 ou directement l'élément d'intervention 13 peut être inséré. De ce fait, l'élément d'intervention 13 et/ou le bras distal 12 de l'élément déformable 10 peut(vent) s'étendre dans l'axe de l'anneau 720 pour aller à une distance plus ou moins proche du cathéter 1.

Ceci constitue un avantage pour permettre une opération d'intervention dans une zone contrôlable visuellement avec des contraintes fortes de précision. Un avantage d'une intervention dans un plan latéral au cathéter 1 est d'obtenir une relative stabilité des éléments d'interventions qui sont solidaires du système d'acquisition de l'image, c'est-à-dire de la sonde échographique de la première fenêtre 3, lorsque le cathéter 1 est introduit.

L'opérateur ou le chirurgien peut, en fonction de sa fenêtre de visualisation, orienter l'élément d'intervention 13 dans le plan du faisceau comme il le souhaite grâce au moyen de pilotage.

Selon un autre mode de réalisation, le premier élément déformable 10 peut être une tige rigide ou souple agencée en périphérie du corps 2 du cathéter 1 comme représenté sur les figures 2E, 2F, 3A à 3D, 4A et 4B.

### Faisceau et élément d'intervention

Lorsque le cathéter 1 est utilisé pour une opération dans le coeur, le faisceau 24 permet de visualiser, dans un plan latéral comprenant l'axe du cathéter 1, l'anneau d'une valve, par exemple, d'une valve mitrale ou tricuspide, les feuillets valvulaire, l'appareil sous valvulaire ainsi que le muscle cardiaque. La visualisation de l'imagerie permet d'obtenir une image extrêmement précise. L'imagerie est alors utilisée pour intervenir localement au moyen d'un élément déformable et d'un élément d'intervention, tel que l'élément d'intervention 13, par exemple, pour fixer une suture sur une partie de la valve, de l'anneau ou des structures anatomiques environnantes.

Un avantage de l'émission du faisceau 24, sensiblement compris dans un plan, est que la zone d'intervention peut être représentée par une image très précise. Cette dernière permet alors d'accompagner une intervention notamment pour une opération nécessitant une précision dans sa manipulation.

Un avantage de l'utilisation d'une sonde échographique combinée au cathéter c'est qu'elle peut être calibrée et configurée pour obtenir une image adaptée à l'opération envisagée. Par exemple, une calibration adaptée à une opération sur une valve mitrale ou tricuspide peut être obtenue avec une portée de faisceau de 1 à 5 cm permettant une manipulation locale de l'élément d'intervention 13 dans une zone proche du cathéter 1.

La figure 2A représente un mode de réalisation dans lequel un second dispositif de guidage angulaire 40 est couplé au cathéter 1. Le cathéter 1 dans un mode de réalisation pourrait n'être équipé que du second dispositif de guidage angulaire 40, quelle qu'en soit la modalité pratique, telle qu'une tige poussoir ou un fil de contrôle, et de la sonde sans pour autant nécessairement comprendre le premier dispositif de guidage angulaire et le premier élément déformable.

Sur cette figure, n'est pas représenté le dispositif de guidage longitudinal 20 de la sonde 23 bien qu'il soit compatible avec ce mode de réalisation.

Dans ce mode de réalisation, le second dispositif de guidage angulaire 40 est fixé par un dispositif de guidage longitudinal 41 sur la surface extérieure du cathéter 1. Ce dispositif de guidage longitudinal peut être par exemple un rail 140 tel que représenté à la figure 2E. L'axe du rail 140 est confondu avec l'axe moyen de la longueur de la fenêtre 3 de sorte à assurer la colinéarité du plan 24 et de l'enveloppe des mouvements du bras mobile distal 42 du dispositif de guidage angulaire et par conséquence de l'élément déformable et de l'élément d'intervention qui seront orientés par le bras mobile distal 42.

Avantageusement, le dispositif de guidage angulaire 40 est amovible et le moyen de pilotage 44 permet de modifier la position longitudinale du dispositif de guidage angulaire 40 par rapport à la fenêtre échographique 3.

Dans cette réalisation, le moyen de pilotage à distance 44 pilote localement les mouvements du bras 41, par exemple sa translation le long du cathéter 1 par exemple si le bras 41 est une tige poussoir. Une particularité de ce mode de réalisation est que le moyen de pilotage à distance 44 permet de contrôler en même temps l'angle d'intervention 48 par la liaison pivot 43. Un avantage est un gain d'encombrement du cathéter 1.

C'est le moyen de pilotage à distance 44 de l'angle d'intervention 48 qui permet d'articuler le bras mobile distal 42 du dispositif de guidage angulaire 40 et d'orienter l'élément déformable et l'élément d'intervention dans le plan du faisceau selon un angle adéquate. Le bras mobile distal 42 sert de support à un élément déformable et/ou un élément d'intervention qui est dirigé dans le plan du faisceau selon l'angle adéquate 48.

Dans un mode de réalisation le bras mobile distal 42 peut être l'élément d'intervention.
Le second dispositif de guidage longitudinal comprend un rail 140 qui est fixé sur la surface extérieur du cathéter 1. Préférentiellement, le rail 140 est colinéaire à l'axe principal de la fenêtre 3. Dans un mode, leurs axes sont confondus comme vu précédemment. Dans un autre mode, le rail est inclus dans le cathéter 1, c'est-à-dire à l'intérieur du cathéter. Ce dernier est alors échancré dans la zone d'intervention du dispositif de guidage angulaire afin de permettre l'utilisation de l'élément d'intervention.

La figure 2E représente un bras rigide 41 translatant sur un rail 140 en forme de « T ». Le rail 140 est solidaire du cathéter 1. Un rail en forme de « T » permet de maintenir parfaitement le second élément déformable dans le plan P.

Un avantage de cet agencement est que les deux éléments d'intervention situés en amont et en aval de la fenêtre 3 évoluent dans le plan du faisceau 24 et permettent par exemple des opérations conjointes de part et d'autre d'une valve. En conséquence, les opérations menées par les éléments d'intervention 13 sont visibles en temps réel sur une fenêtre de visualisation qui récupère les données de la sonde échographique 43.

Ainsi une opération peut être conduite sur une petite partie d'un organe avec une grande précision au moyen de deux éléments déformables et de deux dispositifs d'intervention.

Un avantage est de disposer de deux éléments d'intervention complémentaires, par exemple une pince de préhension permettant d'attraper une partie de l'anneau et une aiguille permettant de percer la partie maintenue au moyen de la pince. Les deux interventions peuvent être contrôlées à tout moment par la sonde échographique 23 qui permet de restituer une image nette des opérations sur un écran. Un autre avantage est de pouvoir reprendre après la ponction le fil de suture ou l'aiguille ayant permis la ponction.

Le cathéter 1 peut comprendre différentes combinaisons d'éléments déformables, de leur type, de leur liaison avec le cathéter 1 et de leur dispositif de guidage angulaire et longitudinal et des dispositifs de maintien utilisés pour stabiliser la sonde ou les éléments d'intervention au besoin.

Les figures 2B à 2D représentent de manière non exhaustive des sections du cathéter 1 ayant différentes combinaisons possibles de dispositifs déformables et de dispositifs de guidage.

La figure 2B représente le cas dans lequel un premier dispositif de guidage longitudinal 74 sous forme d'une gaine ou d'une lumière par laquelle passe(nt) le dispositif de guidage angulaire et/ou l'élément déformable et/ou l'élément d'intervention. Le dispositif de guidage longitudinal 74 comporte un axe se situant dans un plan comprenant le plan du faisceau 24 de manière similaire à la figure 1A. Le cathéter 1 de la figure 2B comprend en outre un rail 140 sur lequel pourra passer le bras principal d'un dispositif de guidage angulaire 40.

La figure 2C représente le cas dans lequel le bras principal, tel que le bras 41, d'un premier dispositif de guidage angulaire et/ou l'élément déformable et /ou l'élément d'intervention translate sur un rail 75 sur la partie du cathéter 1 diamétralement opposée à la fenêtre 3. Lorsque le dispositif de guidage angulaire est utilisé il est agencé le long du rail 75. Un second dispositif de guidage angulaire comprend un bras principal translatant sur un rail 140 sur la partie opposée du cathéter 1, l'axe du rail 140 étant confondu avec l'axe de la fenêtre 3. Le second dispositif de guidage angulaire est alors le long du rail 140, diamétralement opposé au rail 75.

La figure 2D représente le cas dans lequel un premier élément déformable 10 comprend un bras distal 12 agencé à l'intérieur du cathéter 1 et circulant dans un premier dispositif de guidage longitudinal 74 ayant la forme d'une lumière tubulaire 74. L'axe de la lumière tubulaire 74 se situe dans un plan comprenant le plan du faisceau 24. Dans ce mode de réalisation, un second élément déformable, non représenté, par exemple similaire au premier élément déformable 10, est agencé à l'intérieur du cathéter 1 et circule dans un second dispositif de guidage longitudinal 141 ayant également la forme d'une lumière tubulaire 141. L'axe de la lumière tubulaire 141 se situe également dans un plan comprenant le plan du faisceau 24.

La figure 2D représente le cas où il existe une première lumière tubulaire 74 agencée à l'intérieur du cathéter 1 dans laquelle passe un premier dispositif de guidage angulaire et/ou l'élément déformable et/ou l'élément d'intervention. Dans ce mode de réalisation, un second dispositif de guidage angulaire et/ou l'élément déformable et/ou l'élément d'intervention associé est agencé à l'intérieur du cathéter 1 et circule dans une seconde lumière tubulaire 141 coaxiale aux lumières 74 et 21. Les lumières forment chacune un dispositif de guidage longitudinal.

Dans ce mode de réalisation, le cathéter 1 comprend alors une première fenêtre 4 permettant au premier élément d'intervention 13 de s'extraire du cathéter 1 et une seconde fenêtre (non représentée) en amont de la fenêtre 4 permettant à un second élément d'intervention, non représenté, de s'extraire du cathéter 1. Les deux éléments d'intervention sont guidés au moyen d'éléments déformables et/ou de dispositif(s) de guidage angulaire(s) et/ou de dispositif(s) de guidage longitudinal(aux) similaires à ceux précédemment décrits selon des angles d'intervention modifiables.

La figure 2F représente une autre variante de réalisation dans lequel un dispositif de guidage longitudinal 71, de type rail, est agencé à l'intérieur du cathéter 1. Le dispositif de guidage longitudinal 71 permet d'acheminer un bras 77 d'un dispositif de guidage angulaire 7 dans le corps creux 2 du cathéter 1. Le bras principal 120 de l'élément déformable 10 dont le bras distal 12 est guidé par le bras mobile distal 76 du dispositif de guidage angulaire 7. Le bras 77 du dispositif de guidage angulaire 7 est guidé par le dispositif de guidage longitudinal 71 et est mobilisable longitudinalement de telle sorte que sa distance par rapport à la fenêtre 4 est contrôlable par un moyen de pilotage à distance 770 tel qu'une poignée et une vis bloquante par exemple. Le bras 10 est mobilisable longitudinalement et sa distance par rapport à la fenêtre 4 est contrôlable par un dispositif de contrôle à distance 11 telle qu'une poignée et vis bloquante par exemple.

La sonde 23, sa tête 22 et le bras 21 sont représentés sur la figure 2E, en revanche le dispositif de guidage longitudinal 20 n'est pas représenté comme à la figure 1A.

La figure 2G représente deux positions du bras mobile distal 76 du dispositif de guidage angulaire 7 permettant le contrôle d'un angle d'intervention 8. Le bras mobile distal 76 effectue une rotation d'un angle 8 autour d'un axe 78. Le bras 77 peut être un rail de type poussoir effectuant des mouvements de translations 79. La translation du bras 77 permet d'activer la rotation de l'élément 76.

Le bras 77 possède un dispositif de guidage longitudinal pour l'élément déformable 10. Il peut s'agir d'un rail 71 ou d'un simple guide comprenant des arceaux 70 pour maintenir le bras 10.

Dans l'exemple de la figure 2G, le dispositif de guidage angulaire 7 comprend un bras principal 77, un bras mobile distal 76 effectuant le contrôle de l'angle d'intervention 8 et éventuellement des arceaux 70 guidant l'élément déformable 10.

La figure 2F illustre un second élément déformable 10' ayant un bras distal 12' déformable. Le second élément déformable peut être par exemple une gaine à l'intérieur de laquelle un second élément d'intervention 13' est introduit. Dans un autre cas, le second élément d'intervention 13' est fixé à l'extrémité du second élément déformable 10' ou de son bras distal 12'. Le second élément déformable 10' est guidé longitudinalement grâce à un dispositif de guidage longitudinal 70' qui est représenté par des arceaux 70' dans cet exemple de réalisation. Le dispositif de guidage longitudinal 70' est avantageusement solidaire du dispositif de guidage angulaire 40 qui permet d'actionner la rotation d'un bras mobile distal 42. Comme vu précédemment le dispositif de guidage angulaire 40 peut comporter également un dispositif de guidage longitudinal 140 ayant la forme d'un rail.

Selon une alternative, le bras mobile distal 42 peut former un élément déformable voire un élément d'intervention.

Le cathéter 1 de l'invention permet d'offrir différentes combinaisons d'éléments déformables de part et d'autre de la fenêtre 4 selon l'usage envisagé.

Par exemple, le second élément déformable peut être identique au premier élément déformable 10 et être maintenu par un dispositif de guidage angulaire 7 analogue à celui représenté pour le premier élément déformable 10 de la figure 1A. Dans ce dernier cas, deux ouvertures agencées en amont et en aval de la lumière 3 peuvent être conçues de sorte à permettre l'extraction des éléments déformables de type 10 et/ou de leurs bras distaux de type 12' et/ou d'éléments d'intervention de type 13 de part et d'autre du faisceau 23.

Réciproquement, le premier élément déformable peut être du même type que le second élément déformable 10' de la figure 2F, c'est-à-dire fixé sur la surface extérieure du cathéter 1 et guidé par un dispositif de guidage longitudinal de type 70' ou un équivalent et un dispositif de guidage angulaire 40 tel que le dispositif de guidage angulaire guidé longitudinalement par exemple grâce au rail 140. Dans ce dernier cas, deux rails 75 et 140 agencés comme représentés à la figure 2C peuvent être utilisés.

Lorsque deux éléments déformables 10, 10' sont utilisés dans un même cathéter 1, ils sont disposés de part et d'autre de la lumière 3 selon l'axe longitudinal dudit cathéter 1. Dans ce cas, les évolutions des deux éléments d'intervention 13, 13' dans le plan du faisceau 24 peuvent être enregistrées et visualisées sur une imagerie générée à partir des signaux collectés par la sonde échographique 23.

Différents modes de réalisation des éléments déformables 10 et 10', qu'ils soient situés en amont ou en aval de la lumière 3, peuvent être utilisés dans le cathéter 1 de l'invention.

A titre d'exemple, les figures 3A, 3B, 3C, 3D représentent différents modes de réalisation d'un dispositif de guidage angulaire 40 adapté au cathéter 1 de l'invention. Ils peuvent être agencés à l'intérieur ou à l'extérieur du cathéter 1 et comprendre un bras souple ou rigide selon les modes de réalisation de l'invention.

La figure 3A représente un exemple de réalisation du dispositif de guidage angulaire 40. L'extrémité du dispositif de guidage angulaire 40 comprend un bras mobile distal 42 formant un support pour un élément déformable 10' ou un élément d'intervention 13'. Le bras mobile distal 42 est illustré de profil et forme un dispositif basculant. Selon les modes de réalisation, le bras mobile distal peut être considéré comme faisant parti de l'élément déformable ou encore il peut définir l'élément d'intervention selon le cas d'usage envisagé.

Une liaison pivot 43 permet une rotation de l'extrémité du dispositif de guidage angulaire 40 autour d'un axe de rotation fixé au dispositif de guidage angulaire 40. Le bras 41 peut être une tige poussoir qui peut être actionnée par la rotation d'une poignée 44 et au moyen d'une liaison hélicoïdale 47. La liaison hélicoïdale peut être assurée par un système de pas de vis 47. L'élément déformable 10' est maintenu le long du dispositif de guidage angulaire 40 et notamment jusqu'à l'extrémité du bras mobile distal 42 par un ou plusieurs anneaux 70' tels que représentés à la figure 2F.

Avantageusement, lorsque le bras mobile distal est un élément déformable, le dispositif de guidage angulaire 40 et l'élément déformable comportent des éléments en communs, par exemple au niveau et au-delà de la liaison pivot 43. Ce qui est un avantage du point de vue de l'encombrement.

La figure 3B représente la vue de dessus du dispositif de guidage angulaire 40 de la figure 3A lorsqu'il est fixé sur un cathéter 1.

La figure 3C représente une alternative de réalisation de la liaison pivot 43 qui permet le basculement du bras mobile distal 42, dans le plan du faisceau 24. La liaison pivot 430 peut être accompagnée d'une liaison glissière permise par la translation le long du cathéter 1 d'une tige poussoir 41 amovible ou d'une partie seulement de la tige poussoir 41. La translation peut être assurée par une tige évoluant sur un rail fixé à la surface du cathéter 1. La tige poussoir 41 peut être dans ce cas creuse et fendue à son extrémité distale pour former une liaison avec le bras mobile distal 42.

Le rail est alors un dispositif de guidage longitudinal d'un bras d'un dispositif de guidage angulaire 40.

La figure 3D représente une alternative de liaison pivot 431 qui permet le basculement du bras mobile distal 42. La liaison pivot 431 peut être assurée au moyen d'un dégagement latéral des tiges 41, 41' obtenue grâce à un ballon 49 expansible. Le ballon expansible 49 permet la fixation et le maintien des tiges poussoirs 41, 41'. Le ballon expansible 49 est un dispositif de maintien pour stabiliser une position et une orientation. Un tel dispositif peut également être utilisé pour la sonde dans une version sonde amovible du cathéter de l'invention.

Une tige poussoir 41 et une poignée 44 peuvent également être combinées avec un bras souple 21 destiné à maintenir la sonde 23. Ce mode de réalisation n'est pas représenté sur les figures. Par exemple, ce système peut être utilisé pour introduire la sonde 23 dans le cathéter 1 et la positionner au regard de la lumière 3. Pour éviter de générer une différence de longueur entre le bras 21 et le cathéter 1 lorsque la tige poussoir est combinée avec un bras souple 21 représenté à la figure 2, il est possible d'utiliser un dispositif de compensation de longueur.

En effet, un bras souple introduit dans un cathéter 1 peut avoir une courbure différente de celle du cathéter 1 lors de ces déplacements. Dans ce cas, une différence de longueur est constatée à l'extrémité distale ou au niveau de la lumière 3.

Le dispositif de compensation de longueur entre un bras souple et un cathéter 1 permet d'ajuster la position finale par exemple de la sonde 23 en face d'une lumière 3 lorsqu'un tel bras est utilisé avec une sonde amovible. Le dispositif de compensation de longueur peut comprendre par exemple un système d'écrou contre écrou.

Un tel dispositif de compensation de longueur peut également être utilisé avec un dispositif de guidage angulaire fixe ou amovible permettant de contrôler les mouvements d'un élément d'intervention 13.

Lorsque la sonde 23 est positionnée avec la bonne orientation de sorte à être en face de la lumière 3 avec la bonne longueur, un dispositif de maintien peut être utilisé dans le cathéter 1 de l'invention. Par exemple, un dispositif de maintien peut être un ballon agencé à l'extrémité distale du bras 21 permettant de bloquer lorsqu'il se gonfle la sonde 23 dans ses mouvements.

Les figures 4A et 4B représentent deux positions d'une autre alternative d'un dispositif de guidage angulaire combiné à un cathéter 1 de l'invention. Dans ce mode, le dispositif de guidage angulaire comprend une partie 41 à l'intérieur du corps creux 2 du cathéter 1 qui peut être par exemple un fil reliant un moyen de pilotage à distance 44 située sur la partie proximale du cathéter à l'extrémité du bras mobile distal 42 du dispositif de guidage angulaire située sur la partie distale. Le bras mobile distal 42 comprend une extrémité le long de laquelle pourra se positionner un élément déformable, ce dernier pouvant guider le positionnement d'un élément d'intervention à l'extérieur du corps creux du cathéter 1. A titre d'exemple l'élément déformable 10' et l'élément d'intervention 13' de la figure 2F pourraient être employés conjointement à ce mode de réalisation. L'élément déformable 10' est alors maintenu le long et/ou à l'extrémité du bras mobile distal 42 du dispositif de guidage angulaire par un ou plusieurs anneaux tels que les anneaux 70' de la figure 2F.

Ce mode de réalisation est assuré par un moyen de pilotage à distance 44 qui peut être une poignée de contrôle 44. Un fil 41' est alors relié d'une part à la poignée 44 et d'autre part à un bras mobile distal 42 pouvant être par exemple un élément d'intervention ou l'extrémité d'un dispositif angulaire servant de support. Un élément de rappel 45 par exemple formé d'un ressort 45 est assemblé à une liaison pivot 43. L'élément de rappel 45 est solidaire du corps 2 du cathéter 1. Le ressort 45 repousse le bras mobile distal 42 du dispositif de guidage angulaire lorsqu'il est en position permettant d'opérer, c'est-à-dire en position ouverte. La poignée 44 permet soit de libérer le fil pour que le ressort 45 permette le déploiement du bras mobile distal 42, soit de retenir ledit bras mobile distal 42 dans une position de fermeture luttant ainsi contre la force de rappel du ressort 45. En position ouverte, une portion du fil 47 permet d'accompagner le bras mobile distal 42 dans sa rotation. La poignée 44 permet de régler l'orientation du bras mobile distal 42 et donc d'un élément déformable et d'un élément d'intervention utilisés conjointement avec le dispositif de guidage angulaire par un réglage de la portion 47 s'étendant à l'extérieur du cathéter 1.

Avantageusement dans cette solution le fil 41' peut être relié à l'extrémité du bras mobile distal 42 en passant par une fenêtre 4'. Dans cet exemple, la fenêtre 4' peut être située en amont de la fenêtre 3 qui laisse passer le faisceau de la sonde 23, c'est-à-dire que la fenêtre 4' est située sur la partie proximale du cathéter vis-à-vis de la fenêtre 3. Le dispositif de guidage angulaire peut comprendre un élément de maintien 46 permettant d'agir sur le fil 41' de sorte à faciliter sa translation lorsqu'il est extrait du cathéter 1. Ce mode permet d'utiliser le bras mobile distal 42 comme un simple axe ouvert par un ressort et maintenu en position fermée grâce au maintien d'une tension sur le fil 41'. La poignée 44 permet de contrôler l'ouverture et donc l'angle d'intervention 48 du bras mobile distal 42 et donc de l'élément d'intervention.

Un avantage de la solution d'un dispositif de guidage angulaire comprenant un fil, c'est qu'il n'est moins nécessaire de compenser les déformations d'un bras et ses courbures lors de son introduction dans le corps du cathéter 1 lorsque ce dernier est souple. Le fil 41' est déjà en place lors de l'introduction du cathéter 1 et est maintenu tendu au moyen par exemple d'une poignée 44. Lorsque le fil est maintenu tendu, le bras mobile distal 42 reste en position fermée et donc l'élément d'intervention reste également en position fermée.

Un relâchement du fil 41' permet de libérer une portion de fil 47 hors du cathéter 1 lors de l'ouverture du bras mobile distal 42. Un jeu de 1cm ou 2cm de fil supplémentaire permet d'obtenir une ouverture d'angle 48 suffisante pour déployer le bras mobile distal dans le plan du faisceau 24.

Ce mode de réalisation peut être entendu comme un système dit « passif » puisque le l'ouverture du bras mobile distal 42 est effectuée par un ressort et non par une action de l'utilisateur.

Dans une autre variante de réalisation, le ressort 45 permet inversement de maintenir le bras mobile distal 42 en position fermée. L'ouverture du bras mobile distal 42 est effectuée par action sur un fil 41'. L'action peut être par exemple une tension sur le fil vers la direction proximale du cathéter 1.

Un avantage est que le dispositif de guidage angulaire est déjà positionné et peut être correctement orienté par un simple mouvement de la poignée actionnant la tension nécessaire au fil 41'. Ce mode de réalisation ne nécessite pas de réglage de sa position ou de son orientation vis à vis de la fenêtre 3.

Un autre mode de réalisation est la mise en place d'un tel dispositif de guidage angulaire 40 avec un fil de contrôle 41' à l'extrémité d'un bras porteur d'un élément déformable 10 ou 10' qui utilisera le conduit 141 ou 74 du schéma 2D, associé à une ouverture longitudinale en regard de l'extrémité distale 42 du dispositif de guidage angulaire 40 et en amont de la fenêtre 3 échographique.

Un avantage de cette solution est de bénéficier d'une surface lisse du cathéter 1, à l'exception des lumières pour les dispositifs de guidage et les éléments déformables et les éléments d'intervention. Un autre avantage est de pouvoir faire varier la distance de la liaison pivot 43 pour modifier la position de l'élément déformable et/ou de l'élément d'intervention dans le faisceau ultrasonore 24.

Alternativement, le même système est positionné à l'extrémité ouverte d'un tube creux de type une lumière 74 ou 141 souple ou rigide. Par la lumière de ce tube 74 ou 141, un élément déformable 10, 10' est positionnable jusqu'à l'extrémité du dispositif de guidage angulaire 40 ou 7 et au-delà. Cela permet un excellent contrôle de l'élément d'intervention 13, 13' qui est glissé dans l'élément déformable 10 ou 10' lorsqu'il s'agit d'une gaine. Ce tube 74 ou 141 est inclus dans le cathéter 1, et soit fixe, soit mobilisable et contrôlable dans son mouvement longitudinal, par exemple par l'intermédiaire d'une vis bloqueuse, dans une lumière ou sur un rail le long du cathéter 1 en regard de la fenêtre 4 ou en amont ou en aval de la fenêtre échographique 3. Dans ce dernier cas, l'angle d'intervention est alors supérieur à 90 degrés.

Les figures 5A, 5B représentent un mode de réalisation dans lequel la sonde échographique 23 est agencée sur un support 200 pouvant évoluer dans la cavité creuse du corps 2 du cathéter 1. Notamment, selon une variante de réalisation, le support 200 autorise un degré de liberté 61 en rotation de la sonde 23 qui peut émettre un faisceau dans différents plans latéraux au cathéter 1. Le support 200 peut être entendu comme un dispositif de guidage angulaire de la sonde 23. Une configuration de l'agencement de la sonde 23 permet de fixer son orientation de sorte à émettre face à une fenêtre du cathéter 1. Dans ce mode de réalisation, le cathéter 1 comprend une pluralité de fenêtres 401, 402 de sorte à choisir la fenêtre la mieux adaptée à l'enregistrement des actions de l'élément d'intervention, non représenté, guidé par un dispositif de guidage angulaire 40.

Dans ce mode de réalisation, différents éléments déformables peuvent être combinés au cathéter 1 de sorte à limiter l'encombrement de ces derniers autour d'une unique fenêtre.

Le cathéter 1 peut comprendre dans ce cas une pluralité d'éléments déformables de différentes natures qui sont disposés en amont et en aval de différentes ouvertures latérales.

Une simple rotation du cathéter 1 face à l'organe à traiter permet d'offrir différents éléments d'intervention. Une fois le cathéter 1 orienté convenablement, la sonde échographique 23 peut être configurée selon une orientation adaptée à enregistrer les mouvements d'au moins un élément d'intervention.

Par commodité de lecture, le cathéter 1 n'est pas représenté sur la zone du faisceau aux figures 5A et 5B, les ouvertures étant uniquement représentées sur le support 200. De même par commodités, un seul élément déformable est représenté alors que chaque ouverture peut être associée à un tel élément déformable. Selon des variantes de réalisation, le support peut être entièrement ouvert au niveau de la tête de la sonde 23, les ouvertures n'étant alors présentes que sur la surface du corps 2 du cathéter 1.

Lorsque le bras 21 de la sonde échographique 23 est souple, un système d'articulation peut être combiné au bras 21 lui permettant d'actionner une rotation autour de son axe longitudinal. Le système d'articulation peut comprendre par exemple un ou des fils de guidage reliés à l'extrémité distale du bras 21 pour piloter la rotation de la sonde. Un système de roulement peut être utilisé pour faciliter la rotation du bras à partir des fils. Les fils peuvent être au nombre de deux de sorte à engager une rotation dans un premier sens et une rotation dans un second sens.

Les fils peuvent être enroulés sur une roue crantée à l'extrémité proximale, c'est-à-dire proche de la poignée de contrôle du bras. La roue crantée ou tout autre dispositif équivalent permet d'effectuer la rotation nécessaire de la sonde et d'effectuer un serrage des fils de sorte à stabiliser la position de la sonde 23. Une roue crantée peut être combinée à un crochet permettant d'exercer une force pour retenir le fil lorsque ce dernier est enroulé.

Le système d'articulation peut également être appliqué à un élément déformable par exemple pour piloter l'orientation de l'élément d'intervention lorsque ce dernier doit être extrait du cathéter 1 par une fenêtre telle que la fenêtre 4.

La figure 6A représente un coeur humain 80 en coupe comprenant un ventricule gauche 81, un ventricule droite 82, une oreillette gauche 84, une oreillette droite 83. Une valve mitrale 85 est représentée.

La figure 6A permet de mieux comprendre deux types d'interventions qui sont effectuées avec une meilleure précision et une plus grande sécurité pour le patient à partir du cathéter 1 de l'invention.

Une première opération consiste à introduire le cathéter 1 par perforation du ventricule gauche 81 pour venir positionner le cathéter 1 en regard de l'anneau à la base de de la valve mitrale 85. L'introduction du cathéter 1 peut se faire préférentiellement dans la région de l'apex, c'est-à-dire autour de la pointe du ventricule gauche.

Dans cette première opération, un cathéter 1 de l'invention est disposé de sorte à visualiser la valve mitrale 85 ainsi que son anneau et les muscles des parois. Cette première opération permet de d'offrir un champ de visualisation de part et d'autre de la valve 85 tout en permettant une manipulation dans le champ des éléments d'intervention 13 et 13' de part et d'autre de la valve 85.

La première opération consiste à venir positionner la fenêtre 3 du cathéter 1 de manière à visualiser le feuillet valvulaire de la valve 85 dans la zone adéquate à traiter. Un premier 13 et un second 13' élément d'intervention sont déployés dans le plan du faisceau. Le premier élément d'intervention est par exemple une aiguille qui permet le passage d'un fil au travers la valve 85. Ce fil est avantageusement porteur à son extrémité distale d'un dispositif biocompatible l'empêchant de traverser le point de ponction sur le feuillet valvulaire. Le dispositif biocompatible peut être un noeud, un clip, un petit élément volumineux tel qu'une tige, une bille, etc. Le second élément d'intervention 13' permet de récupérer le fil 63 et de le fixer sur un point 62 de la paroi musculaire du coeur. En outre, le fil 63 peut être maintenu en un point 61 de l'extrémité de la valve 85 par exemple par la réalisation d'un noeud ou l'ajout d'un système bloquant le fil tel qu'un clip, une boule de blocage de fil, etc. Avantageusement, le fil possède une aiguille à chaque extrémité et est utilisé pour réaliser à l'aide de deux ponctions un point en U sur le feuillet valvulaire.

La valve est donc maintenue en un point 61 de son extrémité par le fil tendu 63. Le fil 63 une fois maintenu de part et d'autre permet donc un contrôle du mouvement du feuillet valvulaire et par exemple évite un prolapsus de celui-ci.

La première opération a donc nécessité la réalisation d'une perforation du feuillet valvulaire de la valve 85 et le passage d'un fil 63 ainsi que sa fixation sur une paroi du coeur 80.

Une seconde opération peut être réalisée au moyen du cathéter de l'invention. Cette seconde opération est représentée sur le ventricule droit 82. Sa réalisation sur la valve atrio-ventriculaire gauche 85 est similaire.

Le cathéter 1 est inséré dans cet exemple par l'apex au niveau du ventricule droit de manière à le positionner en regard de l'anneau de la valve atrio-ventriculaire 86. L'extrémité distale du cathéter 1 pénètre dans l'oreillette droite de sorte à permettre à un élément d'intervention 13 de perforer l'anneau en un premier point de sa circonférence et y introduire un fil. Le fil est ensuite récupéré par l'élément d'intervention 13' situé dans le ventricule droit.

Dans un second temps, le cathéter 1 effectue une rotation selon l'axe longitudinal du cathéter de sorte à perforer un second point de la circonférence de la base de l'anneau pour y introduire le fil. Ensuite, le fil peut être tendu de sorte à rapprocher le premier et le second points de perforation de sorte à resserrer l'anneau. La seconde opération consiste en un resserrage de la base annulaire. Enfin, une fixation peut être effectuée par exemple au moyen de la réalisation d'un noeud ou de l'ajout d'un clip ou d'un système de fixation des deux fils l'un à l'autre par exemple utilisant un rivet.

La figure 6B représente la base d'un anneau 90 perforé en deux points reliés par un fil.

La figure 6C représente la base de l'anneau 90 après que le fil a été resserré. On comprend que la surface apparente de l'anneau 90 est réduite. Cette seconde opération permet donc de resserrer un anneau, cette opération peut également être entendue comme une annuloplastie d'une valve cardiaque.

Selon d'autres possibilités, d'autres points de perforation peuvent être réalisés dans une opération d'annuloplastie.

Le cathéter 1 peut être combiné avec une ventouse de maintien du coeur. Cette combinaison est particulièrement propice aux opérations qui font intervenir une introduction du cathéter par l'apex du coeur.

Le cathéter 1 comprend dans ce mode de réalisation une ventouse, au centre de la ventouse une ouverture permet l'introduction d'un canal opérateur ne faisant pas perdre à la ventouse sa qualité aspirative et par exemple permet le passage d'un cathéter 1 de l'invention.

L'intérêt majeur de la ventouse est de stabiliser l'apex du coeur, de limiter le risque de saignement et de déchirure de l'apex.

Le système de ventouse peut avantageusement être associé à un dispositif de contrôle des mouvements de la ventouse par sa fixation sur un bras fixe (par exemple fixé à l'écarteur chirurgical ou à la table opératoire). De même, dans cette situation le cathéter 1 est avantageusement relié à un dispositif de contrôle des mouvements longitudinaux au travers de la ventouse et des mouvements angulaires autour du point de ponction sur l'apex du coeur. La mobilisation du cathéter 1 de manière longitudinale et angulaire se fait par l'intermédiaire de trois dispositifs de guidage, l'un contrôlant les mouvements longitudinaux et les deux autres contrôlant la rotation de la partie proximale du cathéter 1 suivant deux arcs de cercles perpendiculaires l'un à l'autre et centrés sur le point de ponction à l'apex du ventricule.

En outre, la présente divulgation concerne une méthode pour opérer une zone du coeur à partir du cathéter 1 de l'invention comme précédemment évoqué à la lumière des figures 6A, 6B, 6C. Cette méthode concerne l'utilisation de l'invention pour la réalisation d'un geste de correction d'une ou des deux valves atrio-ventriculaires cardiaques, également appelées valvules mitrale et tricuspide. L'invention permet notamment la réalisation combinée d'une annuloplastie, correspondant à une plastie de l'anneau, et d'une valvuloplastie, qui correspond à une plastie de la valve proprement dite, c'est-à-dire des feuillets valvulaires et/ou de l'appareil sous valvulaire. L'invention permet également de réaliser de manière indépendante ces deux dernières plasties : plastie de l'anneau d'une part et plastie des feuillets ou de l'appareil sous-valvulaire de la valve d'autre part.

Le but de la méthode est de permettre la réalisation sans difficulté et sans risque de l'annuloplastie et de la plastie du feuillet valvulaire quelle qu'en soit la modalité.

La méthode tire un avantage de l'alignement sur le même instrument des cellules ultrasonores créatrices d'un faisceau ultrasonore et un élément d'intervention basculant dans le plan du faisceau ultrasonore. Il est alors possible de visualiser dans ce faisceau les déplacements de l'élément d'intervention. Ceci permet la visualisation dans le même temps de l'anneau valvulaire avec ses rapports avec les cavités atriales et ventriculaires, ainsi que de l'instrument, tel qu'une aiguille ou une pince par exemple, que l'on désire utiliser au niveau de l'anneau valvulaire.

### Méthodes.

La méthode est compatible avec deux modes opératoires différents. Soit le cathéter de l'invention aborde la valve par voie antégrade atriale, soit par voie rétrograde ventriculaire.

Considérons le premier mode opératoire par l'introduction du cathéter par la voie antégrade atriale. Cette approche peut être soit directe par ponction de la paroi atriale, soit par un accès à distance percutané veineux et ponction transeptal. Dans ce dernier cas, la ponction peut être réalisée au niveau d'une veine périphérique telle que par exemple la veine fémorale ou la veine jugulaire. Cette solution privilégie l'utilisation d'un cathéter de l'invention dans sa version souple.

Dans ce premier mode opératoire, le cathéter 1 atteint la valve mitrale ou tricuspide dans le sens physiologique du flux sanguin.

Lorsque le cathéter atteint l'oreillette, il est poussé sous contrôle scopique ou échographique dans l'orifice valvulaire et stabilisé à ce niveau de manière à pouvoir visualiser l'anneau valvulaire sur l'image échographique qu'il produit. A ce moment, il est possible en utilisant au moins un élément d'intervention du cathéter de l'invention de mettre en place l'instrument qui atteindra l'anneau valvulaire. Le dispositif de l'invention permet également dans son mode privilégié de disposer de deux éléments déformables permettant de mener deux types d'actions simultanément sur l'anneau valvulaire. Selon un cas, un élément d'intervention peut être une aiguille permettant de perforer cet anneau et d'installer une suture transfixiante. Le second élément déformable, tel que 12', rend possible la capture de l'aiguille et/ou de tout élément passé par l'orifice créé par l'aiguille transfixiante. D'autres éléments d'intervention tels que ceux précédemment cités peuvent être utilisés

L'élément d'intervention du cathéter 1 de l'invention peut être situé soit proximalement, soit distalement par rapport au faisceau ultrasonore, soit conjointement de part et d'autre du faisceau ultrasonore. Une fois la première suture mise en place, l'extrémité distale de l'instrument est tournée de quelques degrés de manière à pouvoir mettre en place une seconde suture. Ainsi de proche en proche, il est possible de mettre en place successivement tout au long de l'anneau valvulaire une ou des suture(s) qui peuvent être utilisées pour réaliser l'annuloplastie. L'annuloplastie est concrétisée par une opération de serrage et/ou rapprochement des sutures et/ou en se servant de ces sutures pour fixer un anneau prothétique.

Considérons un second mode opératoire, dans lequel le cathéter 1 est introduit par voie rétrograde.

Cette approche se fait par l'apex (ou par une région proche de l'apex) ventriculaire tel que représenté à la figure 6 sur la paroi latérale du ventricule gauche. Le principe est le même que le premier mode opératoire en sachant que dans ce cas aussi l'élément d'intervention peut se trouver en position proximale ou distale par rapport aux cellules ultrasonores.

Lorsque le cathéter atteint le ventricule, il est poussé sous contrôle scopique ou échographique dans l'orifice valvulaire et stabilisé à ce niveau de manière à pouvoir visualiser l'anneau valvulaire sur l'image échographique qu'il produit. A ce moment, il est possible en utilisant un élément d'intervention du cathéter de l'invention de mettre en place l'instrument qui atteindra l'anneau valvulaire. Comme précédemment l'élément d'intervention peut être par exemple une aiguille permettant de perforer cet anneau et d'installer une suture transfixiante. De même, un second élément déformable, tel que 12', rend possible la capture de l'aiguille et/ou de tout élément passé par l'orifice créé par l'aiguille transfixiante.

D'autres éléments d'intervention tels que ceux précédemment cités peuvent être utilisés. L'élément d'intervention peut être situé soit proximalement, soit distalement par rapport au faisceau ultrasonore généré. Une fois la première suture mise en place, l'extrémité distale de l'instrument tout en restant dans l'orifice valvulaire est tournée de quelques degrés de manière à pouvoir mettre en place une seconde suture. Ainsi de proche en proche il est possible de mettre en place successivement tout au long de l'anneau valvulaire une ou des suture(s) qui peuvent être utilisées pour réaliser l'annuloplastie. Comme énoncé précédemment, l'annuloplastie est concrétisée par une opération de serrage et/ou rapprochement des sutures et/ou en se servant de ces sutures pour fixer un anneau prothétique.

Dans les deux approches, il est possible d'utiliser le cathéter 1 de l'invention pour réaliser un geste diagnostique ou thérapeutique sur les feuillets valvulaires eux-mêmes. Il est ainsi possible de visualiser et d'atteindre à l'aide d'un (des) élément(s) d'intervention du cathéter de l'invention tout point de la valve. L'élément d'intervention selon sa nature permet d'attraper la valve avec une pince, et/ou d'y fixer une suture et/ou d'y réaliser tout geste thérapeutique ou diagnostique. L'ensemble est réalisé avec un contrôle échographique parfait à la fois du positionnement sur le feuillet valvulaire, notamment de la distance entre le bord libre et l'anneau valvulaire et aussi de la profondeur du tissu valvulaire qu'il soit transfixant ou non.

La mise en place d'une ou plusieurs sutures sur l'anneau mitral ou tricuspide à partir du cathéter de l'invention peut également être utilisée pour suturer ou maintenir en place un substitut de remplacement valvulaire introduit par l'abord mini-invasif ou percutané. Cette solution permet de surmonter une problématique majeure causée lors des remplacements valvulaires percutanés : celle d'obtenir un positionnement précis à cheval sur l'anneau valvulaire et d'établir une fixation en place de la valve insérée en percutané.

Par ailleurs, l'invention permet de réduire les fuites paraprothétiques grâce à un maintien correct de la valve en place et par l'application de l'anneau valvulaire natif au substitut prothétique.

Le traitement des valves aortiques et pulmonaires peut également être envisagé avec cette invention en permettant la mise en place précise de points sur l'anneau aortique ou pulmonaire, ainsi que sur la paroi aortique ou pulmonaire.

Le traitement des communications anormales entre les cavités cardiaques peut également être envisagé avec cette invention.

Selon un mode de réalisation de l'invention qui peut se combiner avec tous les précédents modes, les cellules génératrices du faisceau ultrasonographique peuvent être remplacées par une matrice qui génèrera une image 3D volumétrique. Le principe d'imagerie reste analogue à l'obtention d'une image selon un faisceau plan et la méthode de l'invention comprend les mêmes étapes. L'avantage est de permettre une meilleure définition des éléments anatomiques situées autour et à distance du plan de travail de l'élément d'intervention.

## Revendications

1. Cathéter (1) comprenant un corps creux (2) s'étendant longitudinalement selon un axe, comprenant au moins une première fenêtre (3) latérale et une sonde (23) émettant un faisceau (24) d'ondes, ladite première fenêtre (3) latérale permettant le rayonnement du faisceau (24) selon une région latérale au cathéter (1) pour réaliser une imagerie, ladite région latérale étant sensiblement plane selon un premier plan (P1) parallèle à l'axe selon lequel s'étend le cathéter (1), ledit cathéter (1) comprenant en outre un dispositif de guidage longitudinal (140, 70, 70', 71, 74, 141) permettant une transmission d'un mouvement à un élément d'intervention (13, 13'), les déplacements dudit élément d'intervention (13, 13') étant maintenus dans le premier plan (P1), l'angle (8, 48) entre l'élément d'intervention (13, 13') et l'axe du cathéter (1), appelé « angle d'intervention », étant piloté par un moyen de pilotage à distance (73, 44, 770) par un bras mobile distal (72, 76, 42).

2. Cathéter (1) selon la revendication 1, **caractérisé en ce que** la sonde (23) est une sonde échographique ultrasonore et que la fenêtre est une fenêtre ultrasonique, c'est-à-dire ne filtrant pas les ondes ultrasonores.

3. Cathéter (1) selon l'une quelconque des revendications 1 à 2, **caractérisé en ce que** la sonde (23) est capable de se déplacer dans le corps creux (2) et qu'un dispositif de maintien (49) permet d'assurer la stabilité de la sonde (23) lorsqu'elle est positionnée en regard de la première fenêtre (3).

4. Cathéter (1) selon l'une quelconque des revendications 1 à 3, **caractérisé en ce qu'**il comprend un second dispositif de guidage longitudinal (20) permettant de diriger au moins un bras (21) d'une sonde (23) amovible à l'intérieur du corps creux (2) du cathéter (1) et permettant de positionner la sonde (23) en regard de la première fenêtre (3).

5. Cathéter (1) selon l'une quelconque des revendications 1 à 4, **caractérisé en ce qu'**il comprend un élément déformable (10, 10') qui est mobile dans le premier plan (P1) au moyen d'un dispositif de guidage longitudinal (70, 70', 74, 140, 141, 720), l'élément déformable (10, 10') étant adapté à coopérer avec l'élément d'intervention (13, 13') à son extrémité distale.

6. Cathéter (1) selon la revendication 5, **caractérisé en ce que** l'élément d'intervention (13, 13') est fixé et guidé à l'extrémité distale de l'élément déformable (10, 10') par l'intermédiaire d'un bras distal (12, 12').

7. Cathéter (1) selon la revendication 5, **caractérisé en ce que** l'élément déformable (10, 10') comporte une gaine, ledit élément d'intervention (13, 13') étant introduit à l'intérieur de la gaine, l'élément d'intervention (13, 13') et/ou l'extrémité (12, 12') de l'élément déformable (10, 10') étant guidé dans le plan du faisceau (24).

8. Cathéter (1) selon l'une quelconque des revendications 1 à 7, **caractérisé en ce qu'**au moins un dispositif de guidage angulaire (7, 40) est associé à au moins un dispositif de guidage longitudinal (140, 7) de sorte à engager un mouvement de rotation pour piloter l'angle d'intervention (8, 48), le mouvement de rotation étant engagé par un moyen de pilotage à distance (73, 44, 770), ledit mouvement de rotation entraînant la rotation du bras mobile distal (72, 76, 42) du dispositif de guidage angulaire (7, 40) guidant soit l'extrémité distale d'un élément déformable (10, 10'), soit l'élément d'intervention (13, 13'), soit l'ensemble formé de l'extrémité distale de l'élément déformable (10, 10') et de l'élément d'intervention (13, 13').

9. Cathéter (1) selon la revendication 8, **caractérisé en ce que** les moyens de pilotage à distance (44, 73, 770) permettent la conversion d'un mouvement de translation d'un bras (41, 77) du dispositif de guidage angulaire (7, 40) en un mouvement de rotation du bras mobile distal (72, 76, 42), et que le mouvement de translation du bras (41, 77) du dispositif de guidage angulaire (7, 40) est opéré au moyen du dispositif de guidage longitudinal (71, 140, 75).

10. Cathéter (1) selon la revendication 9, **caractérisé en ce que** le dispositif de guidage longitudinal comprend soit une lumière longitudinale (74, 141), soit des arceaux (70, 720, 70'), soit un rail (75, 140, 71), le rail pouvant être à l'intérieur ou à l'extérieur du cathéter (1) et solidaire de ce dernier, que le dispositif de guidage longitudinal (74, 141, 70, 720, 70', 75, 140, 71) permet le guidage longitudinal de tout ou d'une partie du dispositif de guidage angulaire (7, 40) ou d'un élément déformable (10, 10') ou de l'élément d'intervention (13, 13'), ledit dispositif de guidage longitudinal (74, 141) étant associé à son extrémité distale au dispositif de guidage angulaire (7, 40) et étant maintenu dans le premier plan (P1) comprenant l'axe du cathéter (1) et que l'élément déformable (10) est souple et est introduit dans la lumière longitudinale (74, 141) par l'extrémité proximale du cathéter (1), le corps creux (2) du cathéter (1) comprenant une ouverture latérale (4) dont la plus grande dimension est comprise dans le même plan que la plus grande dimension de la première fenêtre (3), ladite ouverture latérale (4) étant adaptée pour permettre les mouvements de rotation du dispositif de guidage angulaire (7, 40) et pour le passage de l'élément d'intervention (13) à l'extérieur du cathéter (1).

11. Cathéter (1) selon l'une quelconque des revendications 5 à 10, **caractérisé en ce que** le cathéter (1) comprend un premier élément déformable (10) associé à un premier élément d'intervention (13), un dispositif de guidage angulaire (7) associé à un dispositif de guidage longitudinal (74, 141, 70, 720, 75), ledit élément déformable (10), ledit dispositif de guidage angulaire (7) et ledit dispositif de guidage longitudinal (74, 141, 70, 720, 75) étant agencés pour que le premier élément d'intervention (13) débouche en aval de la première fenêtre (3) et puisse évoluer dans le plan du faisceau (24) à l'extrémité distale du cathéter (1).

12. Cathéter (1) selon l'une quelconque des revendications 5 à 10, **caractérisé en ce que** le cathéter (1) comprend un premier élément déformable (10) et un second élément déformable (10') associés respectivement à un premier et à un second éléments d'intervention (13, 13'), deux dispositifs de guidage angulaires (7, 40) associés à deux dispositifs de guidage longitudinaux (74, 141, 70, 720, 70', 75, 140), les deux éléments d'intervention (13, 13') débouchant respectivement en aval et en amont de la première fenêtre (3) de sorte à ce qu'un premier élément d'intervention (13) et respectivement un second élément d'intervention (13') puissent évoluer dans le plan du faisceau (24) simultanément ou successivement.

13. Cathéter (1) selon la revendication 1, caractérisé en qu'il comporte au moins trois guides alignés dans le premier plan (P1) colinéaires à l'axe du cathéter (1), un premier guide étant apte à guider longitudinalement la sonde (23) amovible et un second et un troisième guides étant aptes à guider au moins longitudinalement chacun un dispositif de guidage angulaire (7, 40) et/ou un élément déformable (10, 10') et/ou l'élément d'intervention (13, 13'), le premier guide débouchant sur la première fenêtre (3) latérale du corps du cathéter (1), le second guide débouchant en amont de la première fenêtre (3) et le troisième guide débouchant en aval de la première fenêtre (3), le cathéter (1) comprenant en outre au moins un dispositif de guidage angulaire (7, 40) orientable associé à un des second ou troisième guides et dont les mouvements de rotation sont compris dans le premier plan (P1).

14. Cathéter (1) selon la revendication 13, **caractérisé en ce que** le cathéter comprend au moins une ouverture latérale (4) dont la plus grande dimension est comprise dans le même plan que la plus grande dimension de la première fenêtre (3), le second ou le troisième guide débouchant sur l'ouverture latérale (4) du cathéter (1).

15. Cathéter (1) selon l'une quelconque des revendications 1 à 14, **caractérisé en ce que** le cathéter (1) est apte à coopérer avec un introducteur comportant une ventouse à son extrémité proximale pour adhérer par aspiration à un organe à perforer, ledit introducteur comportant un canal opérateur permettant le passage dudit cathéter (1) à l'intérieur dudit organe, ladite ventouse comprenant un canal opérateur central ne communiquant pas avec la partie aspirative de la ventouse.

## Patentansprüche

1. Katheter (1) umfassend einen Hohlkörper (2), der sich in Längsrichtung entlang einer Achse erstreckt, der mindestens ein erstes seitliches Fenster (3) und eine Sonde (23) umfasst, die ein Wellenbündel (24) aussendet, wobei das erste seitliche Fenster (3) die Abstrahlung des Wellenbündels (24) in einen seitlich des Katheters (1) liegenden Bereich ermöglicht, um eine bildliche Darstellung zu erzeugen, wobei dieser seitliche Bereich im Wesentlichen planar über eine erste Ebene (P1) ist, die parallel zur Achse verläuft, entlang der sich der Katheter (1) erstreckt, wobei der Katheter (1) des Weiteren eine Vorrichtung zur Längsführung (140, 70, 70', 71, 74, 141) umfasst, die eine Übertragung einer Bewegung auf ein Interventionselement (13, 13') ermöglicht, wobei die Ortsveränderungen des Interventionselements (13, 13') in der ersten Ebene (P1) gehalten werden, wobei der Winkel (8, 48) zwischen dem Interventionselement (13, 13') und der Achse des Katheters (1), der als "Interventionswinkel" bezeichnet wird, durch eine Fernsteuervorrichtung (73, 44, 770) mittels eines distalen beweglichen Arms (72, 76, 42) gesteuert wird.

2. Katheter (1) gemäß Anspruch 1, **dadurch gekennzeichnet, dass** die Sonde (23) eine Ultraschallsonde ist und dass das Fenster ein Ultraschallfenster ist, das heißt, dass es die Ultraschallwellen nicht filtert.

3. Katheter (1) nach einem der Ansprüche 1 bis 2, **dadurch gekennzeichnet, dass** die Sonde (23) in der Lage ist, sich in dem Hohlkörper (2) zu bewegen, und dass eine Haltevorrichtung (49) es ermöglicht, die Stabilität der Sonde (23) zu gewährleisten, wenn diese gegenüber dem ersten Fenster (3) positioniert ist.

4. Katheter (1) nach einem der Ansprüche 1 bis 3, **dadurch gekennzeichnet, dass** er eine zweite Vorrichtung zur Längsführung (20) umfasst, die es ermöglicht, mindestens einen Arm (21) einer abnehmbaren Sonde (23) im Inneren des Hohlkörpers (2) des Katheters (1) zu lenken und die Sonde (23) gegenüber dem ersten Fenster (3) zu positionieren.

5. Katheter (1) nach einem der Ansprüche 1 bis 4, **dadurch gekennzeichnet, dass** er ein verformbares Element (10, 10') umfasst, das mittels einer Vorrichtung zur Längsführung (70, 70', 74, 140, 141, 720) in der ersten Ebene (P1) beweglich ist, wobei das verformbare Element (10, 10') ausgebildet ist, um mit dem Interventionselement (13, 13') an seinem distalen Ende zusammenzuwirken.

6. Katheter (1) nach Anspruch 5, **dadurch gekennzeichnet, dass** das Interventionselement (13, 13') am distalen Ende des verformbaren Elements (10, 10') mittels eines distalen Arms (12, 12') befestigt und geführt ist.

7. Katheter (1) nach Anspruch 5, **dadurch gekennzeichnet, dass** das verformbare Element (10, 10') eine Hülle aufweist, wobei das Interventionselement (13, 13') in das Innere der Hülle eingeführt wird, wobei das Interventionselement (13, 13') und/oder das Ende (12, 12') des verformbaren Elements (10, 10') in der Ebene des Wellenbündels (24) geführt werden.

8. Katheter (1) nach einem der Ansprüche 1 bis 7, **dadurch gekennzeichnet, dass** mindestens eine Vorrichtung zur winkelförmigen Führung (7, 40) mit mindestens einer Vorrichtung zur Längsführung (140, 7) verbunden ist, so dass eine Drehbewegung bewirkt werden kann, um den Eingriffswinkel (8, 48) zu steuern, wobei die Drehbewegung durch ein Fernsteuerungsmittel (73, 44, 770) bewirkt wird, wobei diese Drehbewegung, die die Drehung des distalen beweglichen Arms (72, 76, 42) der Vorrichtung zur winkelförmigen Führung (7, 40) bewirkt, entweder das distale Ende eines verformbaren Elements (10, 10') oder das Eingriffselement (13, 13') oder die Anordnung aus dem distalen Ende des verformbaren Elements (10, 10') und dem Eingriffselement (13, 13') lenkt.

9. Katheter (1) nach Anspruch 8, **dadurch gekennzeichnet, dass** die Fernsteuerungsmittel (44, 73, 770) die Umwandlung einer Translationsbewegung eines Arms (41, 77) der Vorrichtung zur winkelförmigen Führung (7, 40) in eine Drehbewegung des distalen beweglichen Arms (72, 76, 42) ermöglichen, und dass die Translationsbewegung des Arms (41, 77) der Vorrichtung zur winkelförmigen Führung (7, 40) mittels der Vorrichtung zur Längsführung (71, 140, 75) bewirkt wird.

10. Katheter (1) nach Anspruch 9, **dadurch gekennzeichnet, dass** die Vorrichtung zur Längsführung entweder ein longitudinales Lumen (74, 141), Bögen (70, 720, 70') oder eine Schiene (75, 140, 71) umfasst, wobei sich die Schiene innerhalb oder außerhalb des Katheters (1) befinden und mit diesem fest verbunden sein kann, wobei die Vorrichtung zur Längsführung (74, 141, 70, 720, 70', 75, 140, 71) die Führung in Längsrichtung der gesamten oder eines Teils der Vorrichtung zur winkelförmigen Führung (7, 40) oder eines verformbaren Elements (10, 10') oder des Interventionselements (13, 13') ermöglicht, wobei die Vorrichtung zur Längsführung (74, 141) an ihrem distalen Ende mit der Vorrichtung zur winkelförmigen Führung (7, 40) verbunden ist und in der ersten Ebene (P1) gehalten wird, die die Achse des Katheters (1) umfasst, und wobei das verformbare Element (10) flexibel ist und durch das proximale Ende des Katheters (1) in das longitudinale Lumen (74, 141) eingeführt wird, wobei der Hohlkörper (2) des Katheters (1) eine seitliche Öffnung (4) aufweist, deren größte Abmessung in der gleichen Ebene wie die größte Abmessung des ersten Fensters (3) enthalten ist, wobei die seitliche Öffnung (4) ausgebildet ist, um die Drehbewegungen der Vorrichtung zur winkelförmigen Führung (7, 40) und den Durchgang des Interventionselements (13) außerhalb des Katheters (1) zu ermöglichen.

11. Katheter (1) nach einem der Ansprüche 5 bis 10, **dadurch gekennzeichnet, dass** der Katheter (1) ein erstes verformbares Element (10) umfasst, das mit einem ersten Interventionselement (13) verbunden ist, eine Vorrichtung zur winkelförmigen Führung (7) umfasst, die mit einer Vorrichtung zur Längsführung (74, 141, 70, 720, 75) verbunden ist, wobei das verformbare Element (10), die Vorrichtung zur winkelförmigen Führung (7) und die Vorrichtung zur Längsführung (74, 141, 70, 720, 75) so angeordnet sind, dass das erste Interventionselement (13) dem ersten Fenster (3) nachgelagert mündet und sich am distalen Ende des Katheters (1) in der Ebene des Wellenbündels (24) bewegen kann.

12. Katheter (1) nach einem der Ansprüche 5 bis 10, **dadurch gekennzeichnet, dass** der Katheter (1) ein erstes verformbares Element (10) und ein zweites verformbares Element (10') umfasst, die jeweils einem ersten und einem zweiten Interventionselement (13, 13') zugeordnet sind, zwei Vorrichtungen zur winkelförmigen Führung (7, 40), die zwei Vorrichtungen zur Längsführung (74, 141, 70, 720, 70', 75, 140) zugeordnet sind, wobei die beiden Eingriffselemente (13, 13') jeweils dem ersten Fenster (3) nachgelagert und vorgelagert münden, so dass sich ein erstes Eingriffselement (13) und jeweils ein zweites Eingriffselement (13') gleichzeitig oder nacheinander innerhalb der Ebene des Wellenbündels (24) bewegen können.

13. Katheter (1) nach Anspruch 1, **dadurch gekennzeichnet, dass** er mindestens drei Führungen umfasst, die in der ersten Ebene (P1) kollinear zur Achse des Katheters (1) ausgerichtet sind, wobei eine erste Führung geeignet ist, die abnehmbare Sonde (23) in Längsrichtung zu führen, und eine zweite und eine dritte Führung geeignet sind, jeweils mindestens in Längsrichtung eine Vorrichtung zur winkelförmigen Führung (7, 40) und/oder ein verformbares Element (10, 10') und/oder das Interventionselement (13, 13') zu führen, wobei die erste Führung in das erste seitliche Fenster (3) des Körpers des Katheters (1) mündet, die zweite Führung dem ersten Fenster (3) vorgelagert mündet und die dritte Führung dem ersten Fenster (3) nachgelagert mündet, wobei der Katheter (1) des Weiteren mindestens eine orientierbare Vorrichtung zur winkelförmigen Führung (7, 40) umfasst, die mit einer der zweiten oder dritten Führungen verbunden ist und deren Drehbewegungen innerhalb der ersten Ebene (P1) erfolgen.

14. Katheter (1) nach Anspruch 13, **dadurch gekennzeichnet, dass** der Katheter mindestens eine seitliche Öffnung (4) aufweist, deren größte Abmessung in derselben Ebene enthalten ist wie die größte Abmessung des ersten Fensters (3), wobei die zweite oder die dritte Führung in die seitliche Öffnung (4) des Katheters (1) mündet.

15. Katheter (1) nach einem der Ansprüche 1 bis 14, **dadurch gekennzeichnet, dass** der Katheter (1) geeignet ist, mit einem Einführmittel zusammenzuwirken, das an seinem proximalen Ende einen Saugnapf aufweist, um durch Ansaugen an einem zu perforierenden Organ zu haften, wobei das Einführmittel einen Arbeitskanal aufweist, der den Durchgang des Katheters (1) in das Innere des Organs ermöglicht, wobei der Saugnapf einen zentralen Arbeitskanal aufweist, der nicht mit dem Ansaugteil des Saugnapfs in Verbindung steht.

## Claims

1. A catheter (1) comprising a hollow body (2) longitudinally extending along an axis, comprising at least one first lateral window (3) and a probe (23) emitting a wave beam (24), said first lateral window (3) enabling radiation of the beam (24) along a region lateral to the catheter (1) to perform imaging, said lateral region being substantially planar along a first plane (P1) parallel to the axis along which the catheter (1) extends, said catheter (1) further comprising a longitudinal guide device (140, 70, 70', 71, 74, 141) enabling transmission of a motion to an intervention element (13, 13'), the movements of said intervention element (13, 13') being maintained in the first plane (P1), the angle (8, 48) between the intervention element (13, 13') and the axis of the catheter (1), called an "intervention angle", being driven by a remote drive means (73, 44, 770) by a distal movable arm (72, 76, 42).

2. The catheter (1) according to claim 1, **characterised in that** the probe (23) is an ultrasound probe and **in that** the window is an ultrasonic window, that is not filtering ultrasound waves.

3. The catheter (1) according to any of claims 1 to 2, **characterised in that** the probe (23) is able to move in the hollow body (2) and **in that** a maintaining device (49) enables stability of the probe (23) to be ensured when the latter is positioned facing the first window (3).

4. The catheter (1) according to any of claims 1 to 3, **characterised in that** it comprises a second longitudinal guide device (20) for directing at least one arm (21) of a removable probe (23) inside the hollow body (2) of the catheter (1) and for positioning the probe (23) facing the first window (3).

5. The catheter (1) according to any of claims 1 to 4, **characterised in that** it comprises a deformable element (10, 10') which is movable in the first plane (P1) by means of a longitudinal guide device (70, 70', 74, 140, 141, 720), the deformable element (10, 10') being adapted to cooperate with the intervention element (13, 13') at its distal end.

6. The catheter (1) according to claim 5, **characterised in that** the intervention element (13, 13') is attached and guided at the distal end of the deformable element (10, 10') via a distal arm (12, 12').

7. The catheter (1) according to claim 5, **characterised in that** the deformable element (10, 10') includes a sheath, said intervention element (13, 13') being introduced inside the sheath, the intervention element (13, 13') and/or the end (12, 12') of the deformable element (10, 10') being guided in the plane of the beam (24).

8. The catheter (1) according to any of claims 1 to 7, **characterised in that** at least one angular guide device (7, 40) is associated with at least one longitudinal guide device (140, 7) so as to engage a rotational motion to drive the intervention angle (8, 48), the rotational motion being engaged by a remote drive means (73, 44, 770), said rotational motion causing rotation of the distal movable arm (72, 76, 42) of the angular guide device (7, 40) guiding either the distal end of a deformable element (10, 10'), the intervention element (13, 13'), or the whole formed of the distal end of the deformable element (10, 10') and of the intervention element (13, 13').

9. The catheter (1) according to claim 8, **characterised in that** the remote drive means (44, 73, 770) enable conversion of a translational motion of an arm (41, 77) of the angular guide device (7, 40) into a rotational motion of the distal movable arm (72, 76, 42), and **in that** the translational motion of the arm (41, 77) of the angular guide device (7, 40) is carried out by means of the longitudinal guide device (71, 140, 75).

10. The catheter (1) according to claim 9, **characterised in that** the longitudinal guide device comprises either a longitudinal port (74, 141), archways (70, 720, 70'), or a rail (75, 140, 71), the rail may be inside or outside the catheter (1) and integral with the latter, **in that** the longitudinal guide device (74, 141, 70, 720, 70', 75, 140, 71) enables longitudinal guiding of all or part of the angular guide device (7, 40) or of a deformable element (10, 10') or of the intervention element (13, 13'), said longitudinal guide device (74, 141) being associated at its distal end with the angular guide device (7, 40) and being maintained in the first plane (P1) comprising the axis of the catheter (1) and **in that** the deformable element (10) is flexible and is introduced into the longitudinal port (74, 141) through the proximal end of the catheter (1), the hollow body (2) of the catheter (1) comprising a lateral opening (4) the largest dimension of which is included in the same plane as the largest dimension of the first window (3), said lateral opening (4) being adapted to enable rotational motions of the angular guide device (7, 40) and the intervention element (13) to pass outside the catheter (1).

11. The catheter (1) according to any of claims 5 to 10, **characterised in that** the catheter (1) comprises a first deformable element (10) associated with a first intervention element (13), an angular guide device (7) associated with a longitudinal guide device (74, 141, 70, 720, 75), said deformable element (10), said angular guide device (7) and said longitudinal guide device (74, 141, 70, 720, 75) being arranged so that the first intervention element (13) opens downstream of the first window (3) and can travel in the plane of the beam (24) at the distal end of the catheter (1).

12. The catheter (1) according to any of claims 5 to 10, **characterised in that** the catheter (1) comprises a first deformable element (10) and a second deformable element (10') respectively associated with a first and a second intervention element (13, 13'), two angular guide devices (7, 40) associated with two longitudinal guide devices (74, 141, 70, 720, 70', 75, 140), the two intervention elements (13, 13') respectively opening downstream and upstream of the first window (3) so that a first intervention element (13) and respectively a second intervention element (13') can travel in the plane of the beam (24) simultaneously or successively.

13. The catheter (1) according to claim 1, **characterised in that** it includes at least three guides aligned in the first plane (P1) which are collinear to the axis of the catheter (1), a first guide being capable of longitudinally guiding the removable probe (23) and a second and a third guide being each capable of at least longitudinally guiding an angular guide device (7, 40) and/or a deformable element (10, 10') and/or the intervention element (13, 13'), the first guide opening onto the first lateral window (3) of the body of the catheter (1), the second guide opening upstream of the first window (3) and the third guide opening downstream of the first window (3), the catheter (1) further comprising at least one steerable angular guide device (7, 40) associated with one of the second or third guide and the rotational motions of which are included in the first plane (P1).

14. The catheter (1) according to claim 13, **characterised in that** the catheter comprises at least one lateral opening (4) the largest dimension of which is included in the same plane as the largest dimension of the first window (3), the second or third guide opening onto the lateral opening (4) of the catheter (1).

15. The catheter (1) according to any of claims 1 to 14, **characterised in that** the catheter (1) is capable of cooperating with an introducer including a suction cup at its proximal end to adhere by suction to an organ to be perforated, said introducer including an operating channel for said catheter (1) to pass inside said organ, said suction cup comprising a central operating channel not communicating with the sucking part of the suction cup.
